(19) Europäisches Patentamt / European Patent Office / Office européen des brevets

(11) **EP 4 348 268 B1**

(12) **EUROPEAN PATENT SPECIFICATION**

(45) Date of publication and mention
of the grant of the patent:
**16.04.2025 Bulletin 2025/16**

(21) Application number: **22732782.2**

(22) Date of filing: **25.05.2022**

(51) International Patent Classification (IPC):
***G01N 33/82*** (2006.01)

(52) Cooperative Patent Classification (CPC):
**G01N 33/82;** G01N 2800/042; G01N 2800/044;
G01N 2800/347; G01N 2800/50; G01N 2800/52

(86) International application number:
**PCT/US2022/030935**

(87) International publication number:
**WO 2022/251368 (01.12.2022 Gazette 2022/48)**

(54) **VITAMIN C RENAL LEAK AS A CLINICAL DIAGNOSTIC TOOL IN THE DETECTION, MONITORING, AND MANAGEMENT OF ACUTE AND CHRONIC DISEASES**

NIERENLECKS VON VITAMIN C ALS KLINISCHES DIAGNOSEWERKZEUG BEI DER ERKENNUNG, ÜBERWACHUNG UND VERWALTUNG VON AKUTEN UND CHRONISCHEN ERKRANKUNGEN

PERTE RÉNALE DE VITAMINE C UTILISÉE EN TANT QU'OUTIL DE DIAGNOSTIC CLINIQUE DANS LA DÉTECTION, LA SURVEILLANCE ET LA GESTION DE MALADIES AIGUËS ET CHRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.05.2021 US 202163193012 P**

(43) Date of publication of application:
**10.04.2024 Bulletin 2024/15**

(73) Proprietor: **The United States of America, as represented by
the Secretary, Department of Health and Human Services
Maryland 20892-7788 (US)**

(72) Inventors:
• **LEVINE, Mark A.**
 **Washington, District of Columbia 20015 (US)**
• **EBENUWA, Ifechukwude C.**
 **Washington, District of Columbia 20001 (US)**
• **VIOLET, Pierre-Christian**
 **Bethesda, Maryland 20814 (US)**

(74) Representative: **Maiwald GmbH
Elisenhof
Elisenstraße 3
80335 München (DE)**

(56) References cited:
• **EBENUWA IFECHUKWUDE ET AL: "Abnormal urinary loss of vitamin C in diabetes: prevalence and clinical characteristics of a vitamin C renal leak", AM J CLIN NUTR, vol. 116, no. 1, 10 May 2022 (2022-05-10), pages 274 - 284, XP55951847, Retrieved from the Internet <URL:https://academic.oup.com/ajcn/article-pdf/116/1/274/45085722/nqac063.pdf> DOI: 10.1093/ajcn/**
• **LEVINE M ET AL: "Vitamin C pharmacokinetics in healthy volunteers: evidence for a recommended dietary allowance.", PROCEEDINGS OF THE NATIONAL ACADEMY OF SCIENCES, vol. 93, no. 8, 16 April 1996 (1996-04-16), pages 3704 - 3709, XP55951852, ISSN: 0027-8424, Retrieved from the Internet <URL:https://www.ncbi.nlm.nih.gov/pmc/articles/PMC39676/pdf/pnas01515-0554.pdf> DOI: 10.1073/pnas.93.8.3704**

EP 4 348 268 B1

- CARR ANITRA C ET AL: "Is "renal leak" of vitamin C an issue for people with diabetes?", THE AMERICAN JOURNAL OF CLINICAL NUTRITION, vol. 116, 10 May 2022 (2022-05-10), pages 3 - 4, XP55951850, Retrieved from the Internet <URL:https://academic.oup.com/ajcn/article-pdf/116/1/3/45051229/nqac088.pdf> DOI: 10.1093/ajcn/
- DATABASE Pubmed [online] 1 January 1994 (1994-01-01), SEGHIERI G: "Renal excretion of ascorbic acid in insulin dependent diabetes mellitus - PubMed", XP55952014, retrieved from https://pubmed.ncbi.nlm.nih.gov/7960490/ Database accession no. G Seghieri
- JACKSON JAMES A ET AL: "Screening for Vitamin C in the Urine: Is it Clinically Significant?", JOURNAL OF ORTHOMOLECULAR MEDICINE, vol. 20, no. 4, 1 January 2005 (2005-01-01), pages 259 - 261, XP55951860, Retrieved from the Internet <URL:https://riordanclinic.org/wp-content/uploads/2020/10/Screening-for-Vitamin-C-in-the-Urine-is-it-clinically-significatn-2005-v20n04-p259.pdf>
- LEVINE M ET AL: "Vitamin C pharmacokinetics in healthy volunteers: evidence for a recommended dietary allowance.", PROCEEDINGS OF THE NATIONAL ACADEMY OF SCIENCES, vol. 93, no. 8, 16 April 1996 (1996-04-16), pages 3704 - 3709, XP055951852, ISSN: 0027-8424, Retrieved from the Internet <URL:https://www.ncbi.nlm.nih.gov/pmc/articles/PMC39676/pdf/pnas01515-0554.pdf> DOI: 10.1073/pnas.93.8.3704
- JACKSON JAMES A ET AL: "Screening for Vitamin C in the Urine: Is it Clinically Significant?", JOURNAL OF ORTHOMOLECULAR MEDICINE, vol. 20, no. 4, 1 January 2005 (2005-01-01), pages 259 - 261, XP055951860, Retrieved from the Internet <URL:https://riordanclinic.org/wp-content/uploads/2020/10/Screening-for-Vitamin-C-in-the-Urine-is-it-clinically-significatn-2005-v20n04-p259.pdf>
- DATABASE Pubmed [online] 1 January 1994 (1994-01-01), SEGHIERI G: "Renal excretion of ascorbic acid in insulin dependent diabetes mellitus - PubMed", XP055952014, retrieved from https://pubmed.ncbi.nlm.nih.gov/7960490/ Database accession no. G Seghieri
- HIRSCH I B ET AL: "Ascorbic Acid Clearance in Diabetic Nephropathy", JOURNAL OF DIABETES AND ITS COMPLICATIONS, ELSEVIER SCIENCE, NEW YORK, NY, US, vol. 12, no. 5, 1 September 1998 (1998-09-01), pages 259 - 263, XP027411859, ISSN: 1056-8727, [retrieved on 19980901]
- OREOPOULOS D G ET AL: "Renal excretion of ascorbic acid: effect of age and sex.", JOURNAL OF THE AMERICAN COLLEGE OF NUTRITION., vol. 12, no. 5, 1 October 1993 (1993-10-01), US, pages 537 - 542, XP055952015, ISSN: 0731-5724, Retrieved from the Internet <URL:http://dx.doi.org/10.1080/07315724.1993.10718349> DOI: 10.1080/07315724.1993.10718349
- EBENUWA IFECHUKWUDE ET AL: "Abnormal urinary loss of vitamin C in diabetes: prevalence and clinical characteristics of a vitamin C renal leak", AM J CLIN NUTR, vol. 116, no. 1, 10 May 2022 (2022-05-10), pages 274 - 284, XP055951847, Retrieved from the Internet <URL:https://academic.oup.com/ajcn/article-pdf/116/1/274/45085722/nqac063.pdf> DOI: 10.1093/ajcn/
- CARR ANITRA C ET AL: "Is "renal leak" of vitamin C an issue for people with diabetes?", THE AMERICAN JOURNAL OF CLINICAL NUTRITION, vol. 116, 10 May 2022 (2022-05-10), pages 3 - 4, XP055951850, Retrieved from the Internet <URL:https://academic.oup.com/ajcn/article-pdf/116/1/3/45051229/nqac088.pdf> DOI: 10.1093/ajcn/

**Description**

FEDERALLY SPONSORED RESEARCH OR DEVELOPMENT

This invention was made with Government support under project number Z01-DK053211-15 by the National Institutes of Health, National Institute of Diabetes and Digestive and Kidney Diseases. The Government has certain rights in the invention.

BACKGROUND OF THE INVENTION

Clinical conditions involving the kidney, such as diabetes and Fabry disease, cancers involving the blood or kidney, certain autoimmune diseases (e.g., lupus), cardiovascular disease, and other disorders are often marked by albuminuria and/or proteinuria and also by assessing creatinine and glomerular filtration rate. Albuminuria, proteinuria, creatinine and glomerular filtration rate often serve as clinical markers for such diseases, and the observance of albuminuria/proteinuria in human subjects are often used as a basis for diagnosis, monitoring, and management of such diseases, including the initiation, monitoring, and management of disease treatment. However, albuminuria/proteinuria represents a clinical situation in which either or both the glomerular filtration or protein reabsorption in the proximal tubule is aberrant, significant excess protein (e.g., albumin) is present in the blood or both. In any event, albuminuria/proteinuria represents a situation in which damage has already occurred due to any of various diseases that lead to albuminuria/proteinuria. Thus, albuminuria, proteinuria, creatinine, and glomerular filtration rate are not sufficiently sensitive to assess early-stage disease or renal damage. Jackson et al., Journal of Orthomolecular Medicine, Vol. 20, No. 4 (2005) discuss the clinical significance of screening for vitamin C in urine.

Accordingly, there remains a need for technology to identify and diagnose clinical conditions and risks that can lead to such damage before it occurs, as well as to assist in the monitoring and management of such diseases and other risks of kidney damage and renal impairment.

BRIEF SUMMARY OF THE INVENTION

In a first aspect of the invention, a method of assessing the vitamin C renal leak status of a human subject is provided, wherein the human subject has fasted and not received any supplemental extraneous source of vitamin C for at least six hours and wherein the human subject undergoes an initial micturition after the at least six hours fasting, the initial micturition defining time 0, the method comprising:

(a) collecting all subsequent urine output following time 0 as one or more urine voids from the human subject during a predefined timed interval, wherein the predefined timed interval encompasses a period of time of from 30 minutes to 24 hours and which begins at time 0,
(b) obtaining a plasma sample from the human subject during the predefined timed interval, which begins at time 0,
(c) providing no food or beverage items with high vitamin C content and no supplemental extraneous source of vitamin C (preferably not more than 15 mg vitamin C) throughout the predefined timed interval to the human subject,
(d) assaying for the concentration of vitamin C in the plasma sample,
(e) assaying for the presence of vitamin C in the collected urine output, whereby:
(f) the presence of vitamin C in the collected urine output and a concentration of vitamin C in the plasma sample less than the Minimum Elimination Threshold (MET) indicates the presence of vitamin C renal leak in the human subject, wherein:
(g) the human subject is male, and the MET is between 32.5 $\mu$M and 43.5 $\mu$M, or
(h) the human subject is female, and the MET is between 35.5 $\mu$M and 51.0 $\mu$M.

In an embodiment of the first aspect of the invention, the human subject is male, and the MET is 38.1 $\mu$M or wherein the human subject is female, and the MET is 43.2 $\mu$M.

In another embodiment of the first aspect of the invention, the vitamin C in the collected urine output, in the plasma sample, or both is assayed using liquid chromatography optionally comprising coulometric electrochemical detection with high performance liquid chromatography or chromatography mass spectrometry (LCMS).

In another embodiment of the first aspect of the invention, the human subject does not exhibit albuminuria, proteinuria or both albuminuria and proteinuria.

In a second aspect of the invention, a method of identifying a clinical risk of renal impairment to a human subject is provided comprising assessing vitamin C renal leak in a human subject in accordance with the method of assessing the vitamin C renal leak status of a human subject as described above, whereby an assessment that indicates the presence of vitamin C renal leak in the human subject identifies a clinical risk of renal impairment to the human subject.

In an embodiment of the second aspect of the invention, the human subject exhibits a family history of renal impairment or exhibits a genetic predisposition to develop renal impairment.

In another embodiment of the second aspect of the invention, the clinical risk of renal impairment is due to a disease or an intervention, procedure, or therapy, optionally wherein the intervention, procedure, or therapy comprises administration of a nephrotoxic agent, and optionally wherein the nephrotoxic agent comprises a contrast medium.

In a third aspect of the invention, a method of screening for clinical risk of a disease in a human subject, the method comprising assessing vitamin C renal leak in a human subject in accordance with the method of assessing the vitamin C renal leak status of a human subject as described above, whereby an assessment that indicates the presence of vitamin C renal leak in the human subject supports a conclusion that the human subject is at clinical risk of a disease.

In a fourth aspect of the invention, a method of diagnosing a disease in a human subject is provided, the method comprising assessing vitamin C renal leak in a human subject in accordance with the method of assessing the vitamin C renal leak status of a human subject as described above, whereby an assessment that indicates the presence of vitamin C renal leak in the human subject supports a diagnosis of a disease in the human subject.

In a fifth aspect of the invention, a method of screening for a risk of a disease complication or progression in a human subject is provided, wherein the human subject is a patient with a disease, the method comprising assessing vitamin C renal leak in the human subject in accordance with the method of assessing the vitamin C renal leak status of a human subject as described above, whereby an assessment that indicates the presence of vitamin C renal leak in the human subject supports a conclusion that the human subject is at risk of a disease complication or progression, optionally wherein the disease complication or progression comprises renal impairment.

In a sixth aspect of the invention, a method of monitoring disease state in a human subject is provided, wherein the human subject is a patient with a disease, the method comprising obtaining a first assessment of vitamin C renal leak in the human subject and later obtaining a second assessment of vitamin C renal leak in the human subject, and then comparing the first assessment of renal vitamin C leak with the second assessment of renal vitamin C leak, wherein the renal vitamin C leak is assessed in accordance with the method of assessing the vitamin C renal leak status of a human subject as described above, and wherein:

(i) when the first assessment of vitamin C renal leak does not indicate the presence of renal vitamin C leak in the human subject and the second assessment of vitamin C renal leak indicates the presence of vitamin C renal leak in the human subject, a determination of disease progression in the human subject is made, or wherein

(ii) when the first assessment of vitamin C renal leak does not indicate the presence of renal vitamin C leak in the human subject and the second assessment of vitamin C renal leak does not indicate the presence of renal vitamin C leak in the human subject, a determination of disease stasis in the human subject is made, or wherein

(iii) when the first assessment of vitamin C renal leak indicates the presence of renal vitamin C leak in the human subject and the second assessment of vitamin C renal leak does not indicate the presence of vitamin C renal leak in the human subject, a determination of disease regression in the human subject is made, or wherein

(iv) when the first assessment of vitamin C renal leak indicates the presence of renal vitamin C leak in the human subject and the second assessment of vitamin C renal leak indicates the presence of renal vitamin C leak in the human subject, a determination of disease stasis in the human subject is made.

In a seventh aspect of the invention, a method of predicting the response of a human subject to therapeutic treatment or prophylaxis is provided, the method comprising assessing vitamin C renal leak in a human subject, wherein the human subject is a patient in need of therapeutic treatment of or prophylaxis from a disease, wherein the vitamin C renal leak is assessed in accordance with the method of assessing the vitamin C renal leak status of a human subject as described above, wherein an assessment that indicates the presence of vitamin C renal leak in the human subject results in a prediction that the human subject will be responsive to the therapeutic treatment or prophylaxis.

In an embodiment of the seventh aspect of the invention, the therapeutic treatment or prophylaxis comprises a procedure or agent bearing a risk of renal impairment.

In another embodiment of the seventh aspect of the invention, the therapeutic treatment or prophylaxis comprises the administration of an angiotensin converting enzyme (ACE) inhibitor, an aldosterone receptor blocker (ARB), an antihyperglycemic agent, a GLP-1 agonist. an SGLT-2 inhibitor, enzyme-replacement therapy, or vitamin C.

In an embodiment of the third to seventh aspect of the invention, the disease is diabetes, an etiology involving the kidney, an autoimmune disease involving the kidney, a chronic urinary tract infection, Fabry disease, Retinopathy, cardiovascular disease, stroke, cancer, or HIV disease.

In another embodiment of the third to seventh aspect of the invention, the diabetes is type 1 diabetes or type 2 diabetes.

In another embodiment of the first to seventh aspect of the invention, the methods comprise further assessing fractional excretion of vitamin C (FeVC) in the human subject, comprising

(i) assaying for the concentration of vitamin C in the collected urine output,
(j) assaying for the volume of the collected urine output,
(k) assaying for the concentration of creatinine in the collected urine output, and
(l) assaying for the concentration of creatinine in the plasma sample,
(m) wherein the FeVC value is calculated as "A" according to the following equation:

$$A = \frac{\dfrac{[urine\ vitamin\ C\ concentration\ (\mu M)] * [urine\ volume\ (L)]}{[plasma\ vitamin\ C\ concentration\ (\mu M)]}}{\dfrac{[urine\ creatinine\ concentration\ (\mu M)] * [urine\ volume\ (L)]}{[plasma\ creatinine\ concentration\ (\mu M)]}}$$

BRIEF DESCRIPTION OF THE SEVERAL VIEWS OF THE DRAWING(S)

Figure 1A is pertinent to Example 1 and depicts the enrollment and patient flow for the vitamin C depletion-repletion study.

Figure 1B depicts the enrollment and patient flow for the vitamin C renal leak study in men with Fabry disease and healthy male controls.

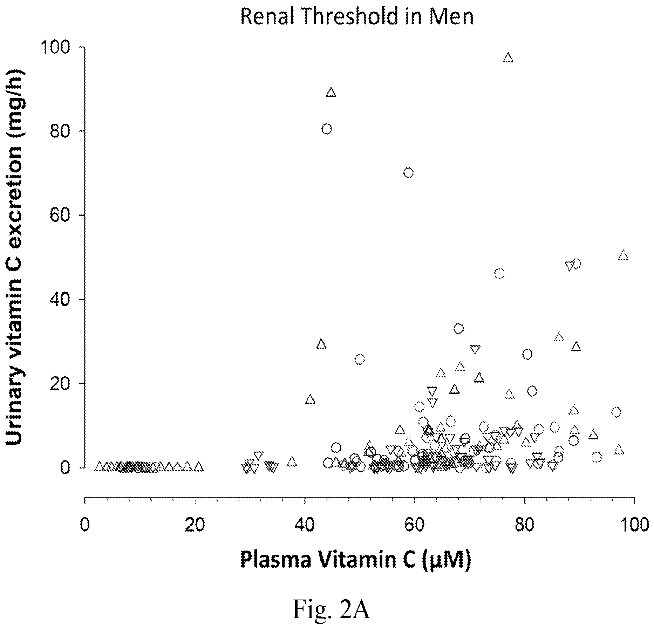

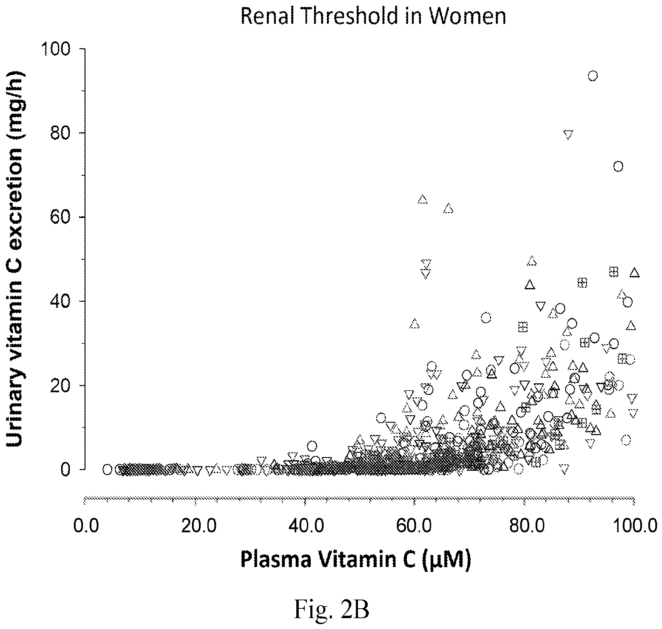

Figures 2A-2D present data pertinent to Example 1 from the vitamin C depletion-repletion study in healthy subjects. Figure 2A graphically presents data depicting urinary vitamin C excretion as a function of matched plasma vitamin C concentrations in healthy men. Figure 2B presents data depicting urinary vitamin C excretion as a function of matched plasma vitamin C concentrations in healthy women. Pharmacokinetic studies (PK) were performed by administering intravenous or oral doses (15-1250 mg) of vitamin C on successive days at steady-state. Blood samples were obtained at fixed intervals. All urine voided during the test period was collected, and each urine void formed a timed collection. The total amount of vitamin C in each urine sample (mg) is indicated on the y axis. Each symbol shape coupled to a specific fill pattern represents a different subject. Data are shown from 10-14 PK studies (5-7 oral doses; 5-7 intravenous doses) for each subject. Participants were 7 men and 10 women.

Figure 2C graphically presents data depicting estimated vitamin C renal threshold values in healthy men and women using physiology-based pharmacokinetic modeling (PBPK, ■) and population curve fitting (PCF, □).

Figure 2D graphically presents data concerning vitamin C renal leak criteria for men (□) and women (■). Minimal Elimination Threshold (MET) was defined as 2SD below mean renal threshold using PBPK (see Example 1 below, methods section). Vitamin C renal leak was defined as inappropriate urinary vitamin C excretion (>0.01mg/total urine volume, limit of detection) occurring when plasma vitamin C concentrations were less than MET values of 38.11$\mu$M for men and 43.2$\mu$M for women.

Figures 3A-3F depict data pertinent to Example 1 from the Vitamin C renal leak study in healthy men and men with Fabry disease.

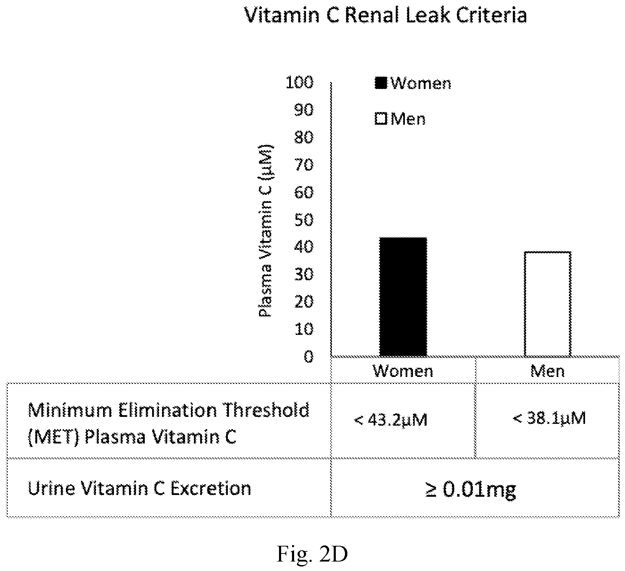

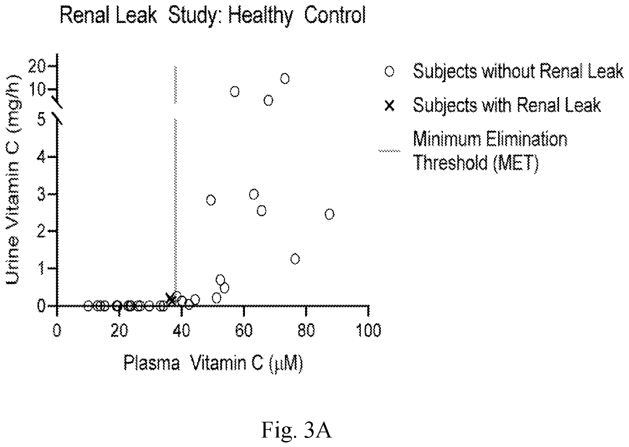

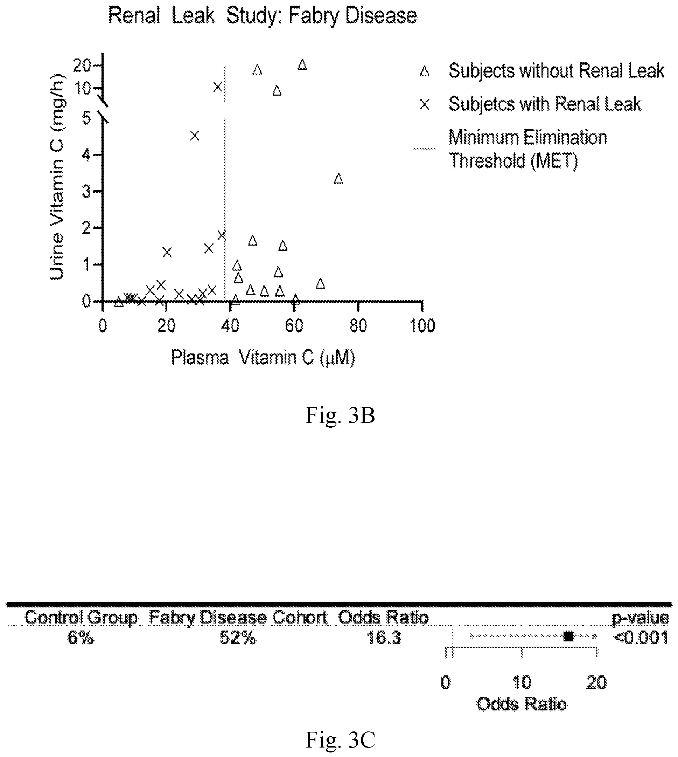

Figure 3A graphically presents data for urinary vitamin C excretion as a function of plasma vitamin C concentrations in healthy men (○). Figure 3B graphically presents data for urinary vitamin C excretion as a function of plasma vitamin C concentrations in men with Fabry disease (△). The vertical dash-line indicates the Minimal Elimination Threshold (MET) (see figure 2D). For Figures 3A and 3B, subjects with urinary vitamin C excretion less than the MET are classified as having a renal leak (×). A one-hour timed urine collection and a matched fasting blood sample was obtained from each subject. Each symbol represents a different single subject.

Figure 3C graphically presents data depicting vitamin C renal leak prevalence in Fabry's disease compared to healthy controls. The analyses is derived from data in Figure 3A and Figure 3B.

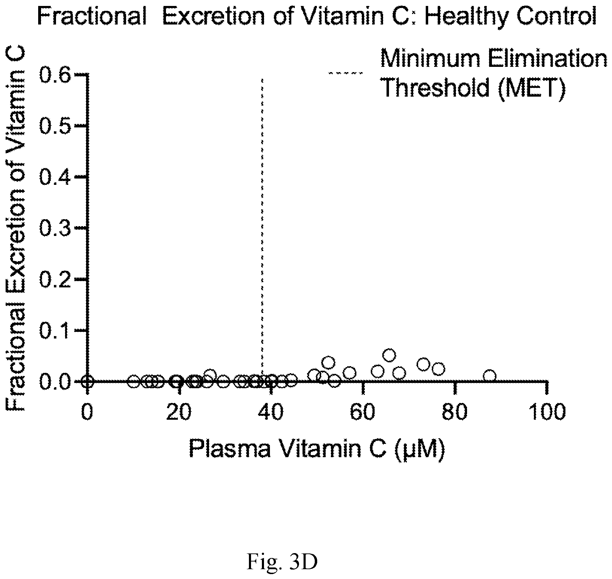

Figure 3D graphically presents data concerning the fractional excretion of vitamin C (FeVC) as a function of plasma vitamin C concentrations in healthy men (O). The vertical dashed line indicates the Minimal Elimination Threshold (MET).

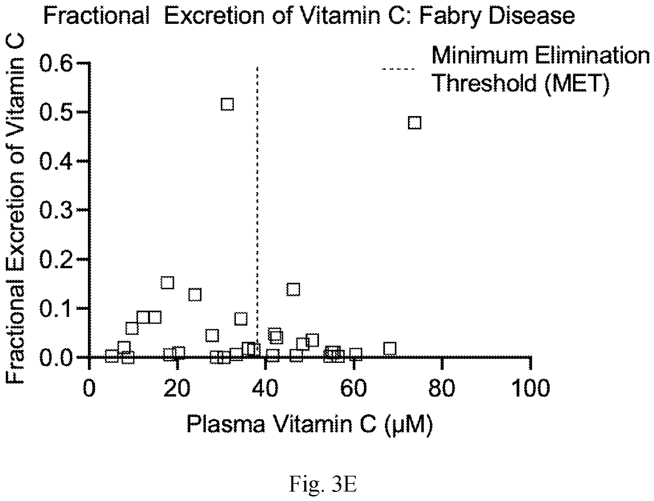

Figure 3E graphically presents data concerning the fractional excretion of vitamin C (FeVC) as a function of plasma vitamin C concentrations in men with Fabry disease (□). The vertical dashed line indicates the Minimal Elimination Threshold (MET).

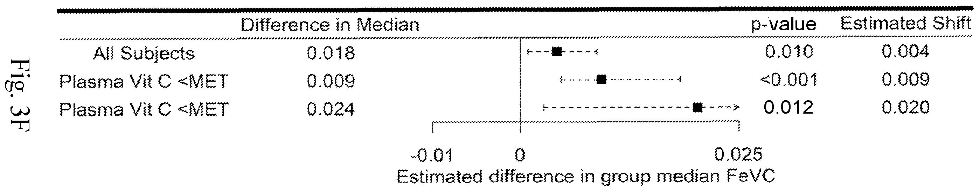

Figure 3F presents data concerning the median FeVC in Fabry disease compared with healthy control for all subjects, and for subgroups with plasma vitamin C less than the MET and greater than the MET. Shift in median represents the difference of median FeVC between the two groups. Analyses derived from data in Figures 3D and 3E.

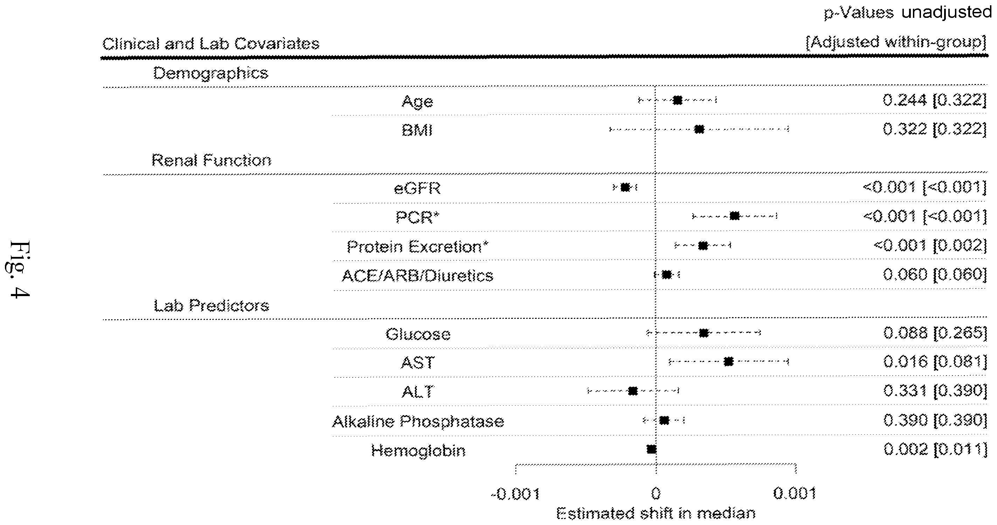

Figure 4 graphically presents data pertinent to Example 1 concerning clinical variables and their predictive association with vitamin C renal leak expressed as fractional excretion of vitamin C (FeVC). Squares indicate estimated shift in Box-Cox transformed means, with horizontal points indicating 95% CIs. Some values were adjusted to fit the scale of the forest plot. *PCR and Protein expression values were multiplied by 100. * * ACE/ ARB/Diuretics and hemoglobin value were divided by 10. Raw values are displayed in Table 3. Abbreviations: BMI, body mass index; PCR, protein creatinine ratio; ACE, angiotensin-converting enzyme inhibitor; ARB, angiotensin receptor blocker; AST, aspartate aminotransferase; ALT, alanine aminotransferase.

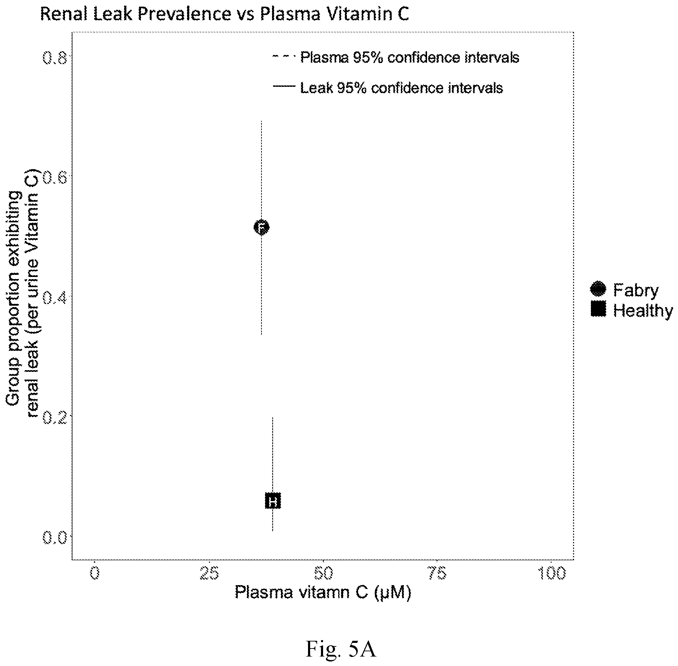

Figures 5A-5C present data pertinent to Example 1 concerning vitamin C renal leak as a function of plasma vitamin C concentrations in men with Fabry Disease and healthy male controls.

Figure 5A graphically presents data concerning vitamin C renal leak using 1h timed urine collection, as a function of plasma vitamin C concentrations in healthy men and men with Fabry disease.

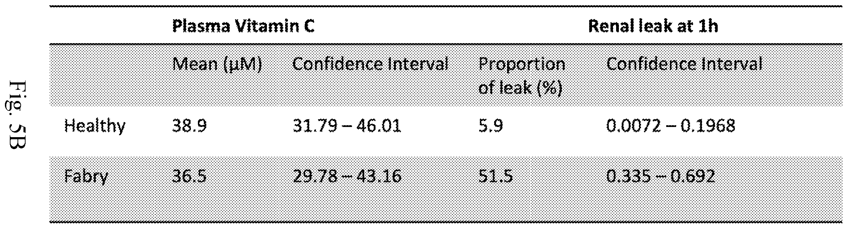

Figure 5B presents the numerical values for the data depicted in Figure 5A. Following a 3-week vitamin C restricted diet by the healthy controls but not Fabry cohort, the mean plasma vitamin C concentrations in both groups were not statistically different (p-value = 0.614).

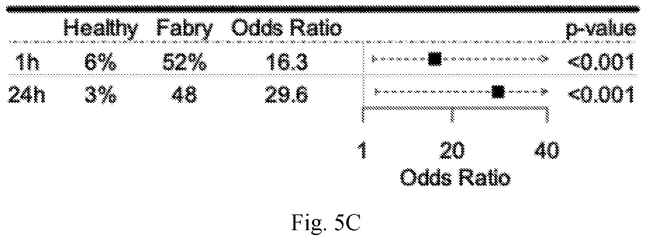

Figure 5C presents data concerning Vitamin C renal leak prevalence in Fabry disease compared to healthy controls at 1h and 24h urine collection.

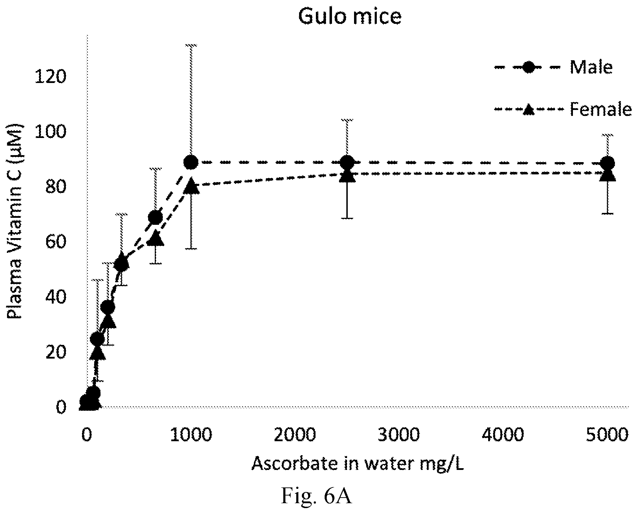

Figures 6A-6D present data pertinent to Example 1 concerning plasma and urine vitamin C concentrations in gulo$^{-/-}$ mice and SVCT1$^{-/-}$ mice supplemented with vitamin C in drinking water. Values represent means +/- SDs. All mice were maintained at each dose for one week before blood and urine sampling. Vitamin C was added to drinking water at amounts of 0 to 5000mg/L.

Figure 6A graphically presents data concerning plasma and urine vitamin C concentrations in female (▲) and male (●) gulo$^{-/-}$ mice as a function of vitamin C amount in drinking water. Values represent means +/- SDs.

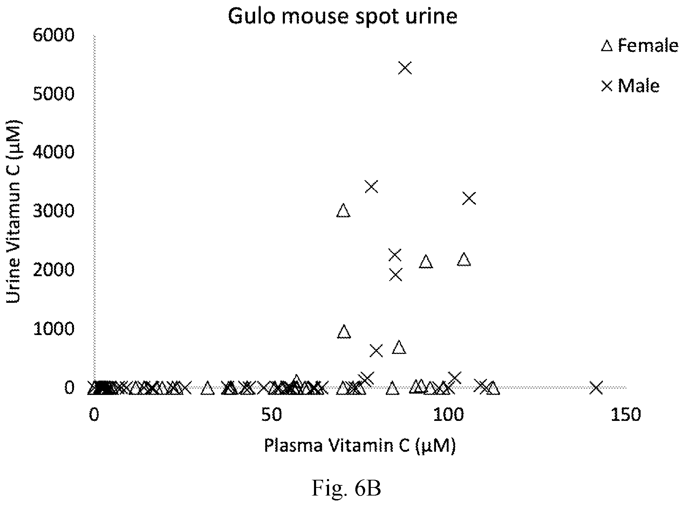

Figure 6B graphically presents data concerning plasma vitamin C concentrations in relation to urine concentration of vitamin C in female (△) and male (×) gulo$^{-/-}$ mice. Each symbol represents one plasma and urine sample obtained at the same time from one mouse at one dose.

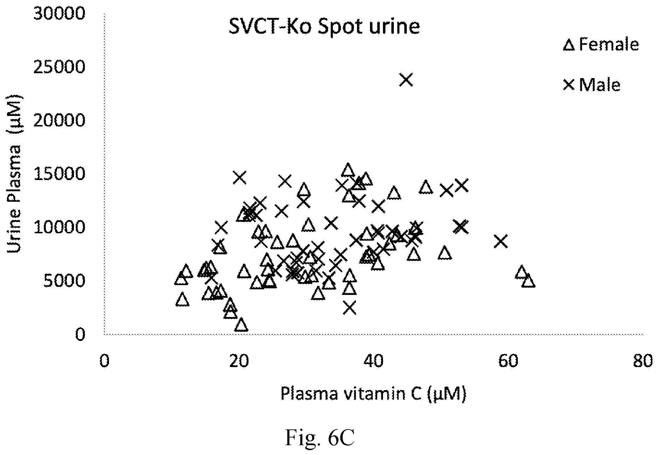

Figure 6C graphically presents data concerning plasma vitamin C concentrations in relation to urine concentrations of vitamin C in female (△) and male (×) SVCT1$^{-/-}$ mice (SVCT1-Ko). Each symbol represents one plasma and urine sample obtained at the same time from one mouse at one dose.

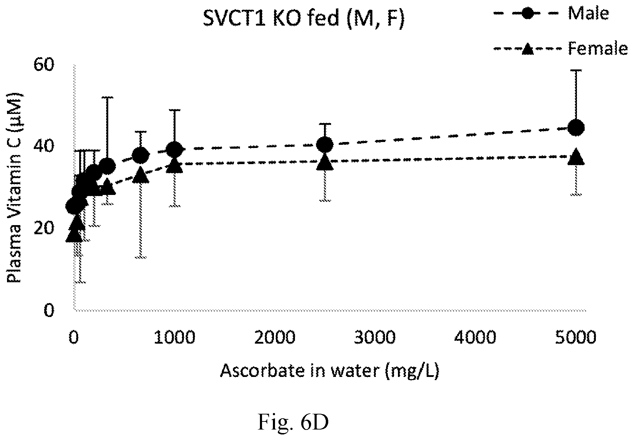

Figure 6D graphically presents data concerning Plasma and urine vitamin C concentrations in female (▲) and male (●) SVCT1$^{-/-}$ mice (SVCT KO) as a function of vitamin C amount in drinking water. Values represent means +/- SDs.

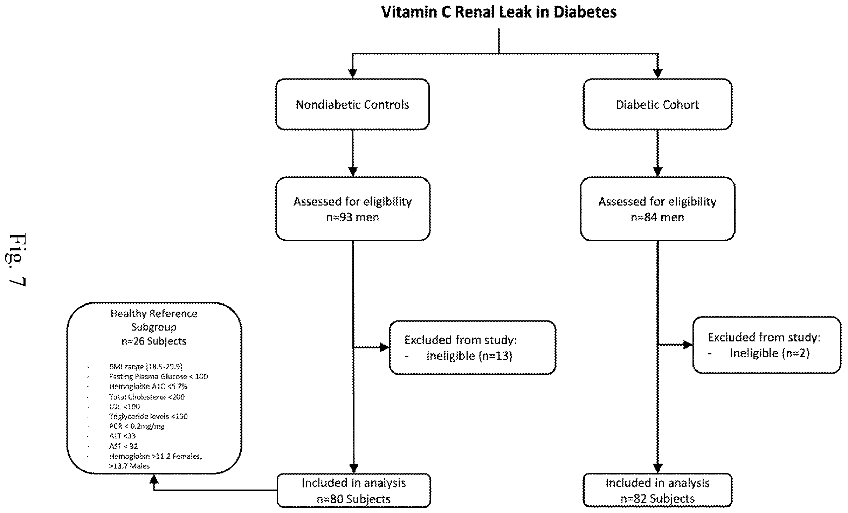

Figure 7 is pertinent to Example 2 and depicts the enrollment and patient flow for participants in the diabetes cohort, nondiabetic controls and healthy subgroup. The healthy subgroup is a subset of the nondiabetic controls whose laboratory values were within the reference ranges.

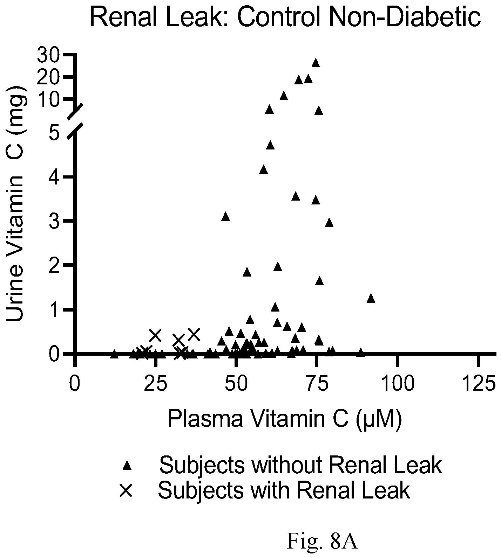

Figures 8A-8E present data pertinent to Example 2 concerning Vitamin C renal leak prevalence in the diabetic cohort, nondiabetic controls, and healthy subgroup. Numeric data are presented in Table 5.

Figure 8A graphically presents data concerning urinary vitamin C excretion as a function of plasma vitamin C concentrations in the nondiabetic control group (▲). Subjects with urinary vitamin C excretion less than the MET are classified as having a renal leak (×). A one-hour timed urine collection and a matched fasting blood sample was obtained from each subject. Each symbol represents a different single subject.

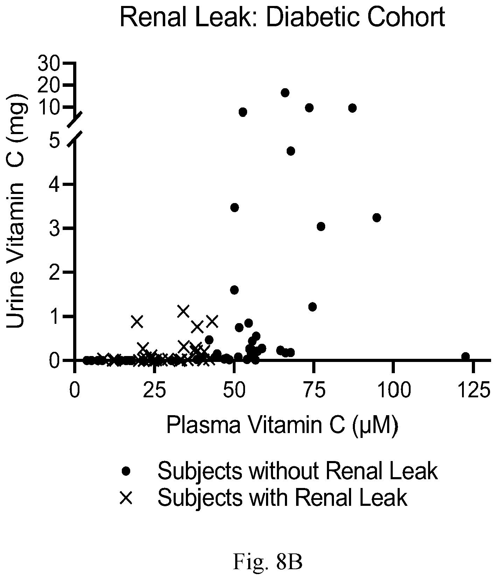

Figure 8B graphically presents data concerning urinary vitamin C excretion as a function of plasma vitamin C concentrations in the diabetic cohort (●). Subjects with urinary vitamin C excretion less than the MET are classified as having a renal leak (×). A one-hour timed urine collection and a matched fasting blood sample was obtained from each subject. Each symbol represents a different single subject.

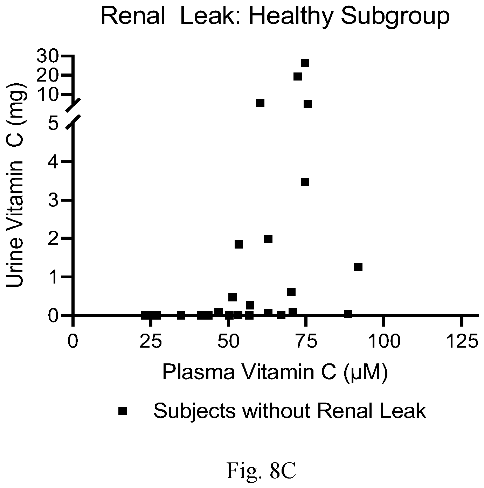

Figure 8C graphically presents data concerning urinary vitamin C excretion as a function of plasma vitamin C concentrations in the healthy subgroup (■). Subjects with urinary vitamin C excretion less than the MET are classified as having a renal leak (×). A one-hour timed urine collection and a matched fasting blood sample was obtained from each subject. Each symbol represents a different single subject.

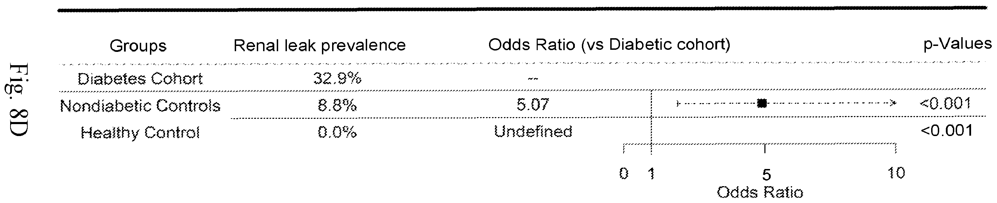

Figure 8D presents data concerning the vitamin C renal leak prevalence in diabetic cohort compared with nondiabetic controls and healthy subgroup.

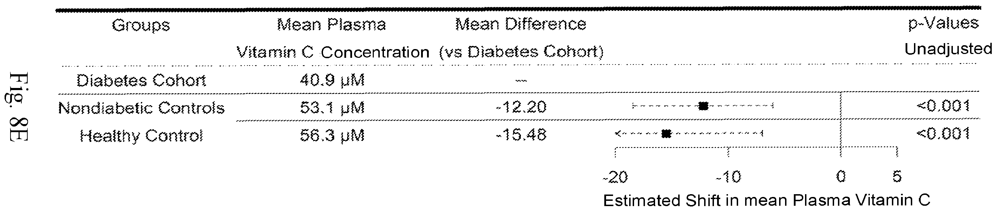

Figure 8E presents data concerning the plasma vitamin C concentrations in the diabetic cohort compared with nondiabetic controls and healthy subgroup.

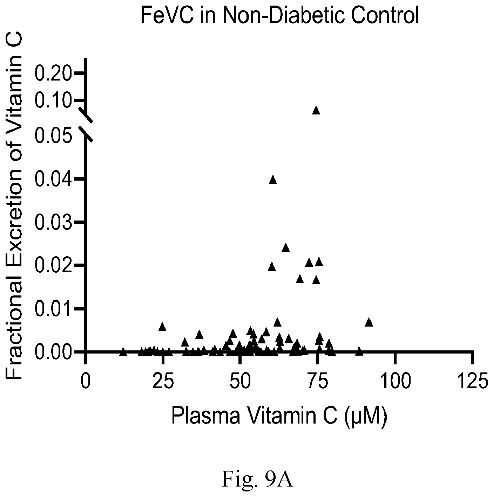

Figures 9A-9D present data pertinent to Example 2 concerning Fractional Excretion of Vitamin C (FeVC) in the diabetic cohort, nondiabetic controls and healthy subgroup. Numeric data are presented in Table 6.

Figure 9A graphically presents data concerning FeVC as a function of plasma vitamin C concentrations in the nondiabetic control group (▲).

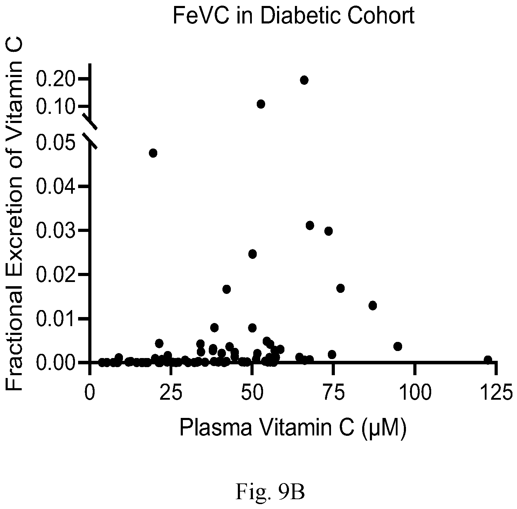

Figure 9B graphically presents data concerning FeVC as a function of plasma vitamin C concentrations in the diabetic cohort (●).

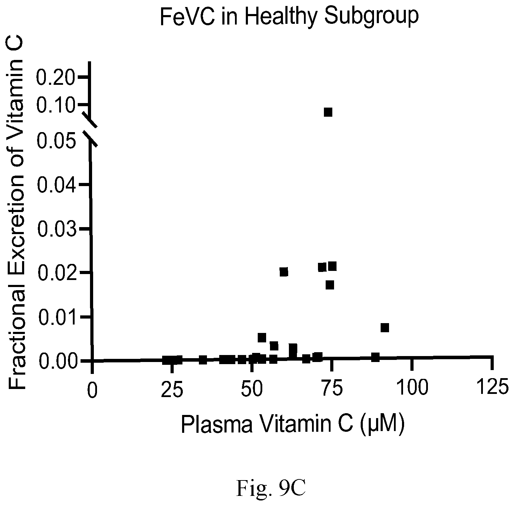

Figure 9C graphically presents data concerning FeVC as a function of plasma vitamin C concentrations in the healthy subgroup (■).

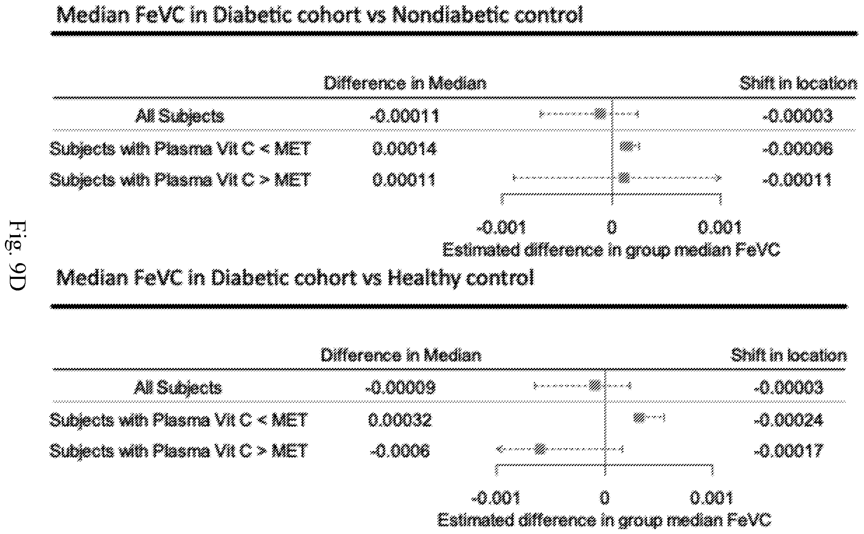

Figure 9D presents data concerning the median FeVC in diabetic cohort, nondiabetic control and healthy subgroup. Comparison was done for all subjects and subgroups with plasma vitamin C concentrations less than, and greater than the MET. Shift in location represents the difference of median FeVC between the groups.

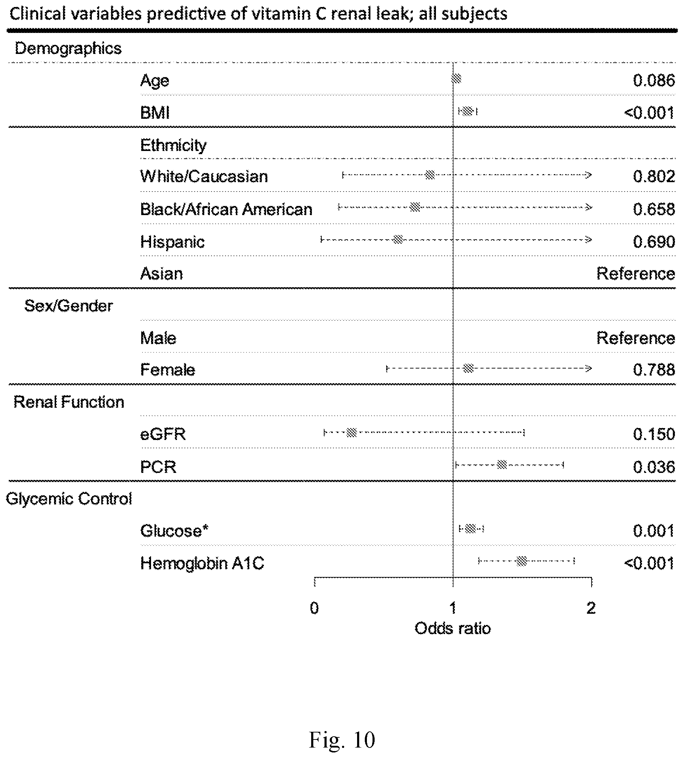

Figure 10 presents data pertinent to Example 2 concerning clinical variables and their predictive association with vitamin C renal leak. All subjects were included in the analysis. Squares indicate odds ratio with horizontal points indicating 95% CIs. Some values were adjusted to fit the scale of the forest plot. *Glucose and Age values were multiplied by 10. Raw values are displayed in Table 7. Abbreviations: BMI, body mass index; eGFR, estimated glomerular filtration; PCR, protein creatinine ratio.

DETAILED DESCRIPTION OF THE INVENTION

In accordance with the present invention, a human subject is assessed for the presence of vitamin C renal leak. The method of the invention in its general sense is defined by independent claim 1. The human subject can be male or female and can be a patient with a disease or a subject that has no diagnosis of disease. In the context of the present invention, the human subject should be at least one week old, but can be a child, an adolescent, or an adult (at least 18 years of age). In certain embodiments, the human subject does not exhibit albuminuria, proteinuria or both albuminuria and proteinuria; however, the inventive method is not limited in its application to such human subjects not exhibiting albuminuria, proteinuria, or, indeed, any other marker of disease.

To assess vitamin C renal leak, the human subject first undergoes a period of fasting, typically lasting at least six hours (or at least about six hours), preferably from eight to twelve hours. The period of fasting could differ from six hours; for example, if the human subject has consumed or other received a high amount of a supplemental extraneous source of vitamin C (e.g., 1 g, such as in a dietary supplement), a fast of about eight hours or longer may be desired. Preferably, the period of fasting includes an overnight fast. During the fast, the human subject consumes no food and receives no other supplemental extraneous source of vitamin C (such as a multivitamin, tea, or other non-food supplement (e.g., intravenous fluid) containing vitamin C). During the period of fasting, the subject can receive water if desired, and medications, however. The purpose of the fast is to prevent dietary or other intake of vitamin C from elevating the plasma or urine vitamin C concentration, which is the subject of the inventive assessment of vitamin C renal leak.

After the human subject has fasted for at least six hours (or at least about six hours), the human subject undergoes an initial micturition. The purpose of this initial micturition is to clear the bladder of urine that represents renal filtration during the at least six-hour period of fasting, which may otherwise contribute to vitamin C in the human subject's plasma and/or urine. The initial micturition can be said to define a start time (time 0) of a predefined timed interval, during which fluid samples are obtained from the fasting human subject as described herein. The predetermined timed interval can span any suitable length of time, such as 30 minutes (or about 30 minutes), 60 minutes (or about 60 minutes), 90 minutes (or about 90 minutes), about one or several hours, and up to and including 24 hours (or up to and including about 24 hours). Throughout the predefined timed interval, the human subject is not provided food or beverage items with high vitamin C content (such as, but not limited to orange juice or citrus fruit) or any supplemental extraneous source of vitamin C (such as, but not limited to, an oral vitamin C or multivitamin supplement or intravenous source of vitamin C). Preferably, the human subject receives not more than 15 mg vitamin C during the predefined timed interval. The human subject can otherwise be provided with food, water, and medications during the predefined timed interval.

After the initial micturition (time 0), the assessment of vitamin C renal leak proceeds by collecting all subsequent urine output from the human subject during the predefined timed interval, which begins at time 0. The urine output is collected from the human subject during this predefined timed interval, which begins at time 0, as one or more urine voids. The urine voids from the human subject during the predefined timed period are combined into one volume (collected urine output) for assessment in accordance with the inventive method. Vitamin C renal leak can be assessed using a block-time urine collection coupled to a plasma sample as well as a full 24-hour urine collection coupled to a plasma measurement. Block-time measures, over 30 or 60 minutes for example, are convenient and feasible in outpatient settings, especially when coupled to an overnight fast to avoid biased estimates from vitamin C ingestion within the prior 8-12 h. Urine collections for 24 hours may be less preferable for several reasons, not merely inconvenience. Because they are usually performed at home, there is potential for incomplete collection due to inadvertent error. There may be confounding data in subjects who have plasma concentrations slightly below the Minimum Elimination Threshold (MET) (discussed herein) but who consume vitamin C-enriched juices or foods during the 24 hours of sample collection. The urine is collected from the human subject using standard clinical laboratory-accepted methodology, e.g., using a collection cup, tray, urinal, etc., and may be aided using a catheter, cannula, or other similar device.

Also, during the predefined timed interval, which begins at time 0, a plasma sample is collected from the human subject. Multiple plasma samples can, if desired, be taken and could be helpful but are generally not feasible in an outpatient setting. To collect the plasma sample, blood is drawn from the human subject using standard clinical laboratory-accepted methodology (e.g., using a venous blood draw (such as using a heparin tube) and collection tube or bag) and, if desired, can be processed using standard methods to separate blood components from plasma.

Thus, in performing the inventive method the urine output and the plasma sample are collected from the fasting human subject during the same predefined timed interval, which begins at time 0. This timing ensures that the urine output collected from the human subject and the plasma sample obtained from the human subject are reflective of a common physiological and nutritional vitamin C state vis-à-vis the human subject.

Following collection, the plasma sample is assayed to assess the concentration of vitamin C in the plasma sample. Similarly, following collection, the collected urine output is assayed to assess the presence of vitamin C in the collected urine output. Other parameters, such as the volume of either or both of the collected urine output and plasma sample and the absolute amount of vitamin C present in the collected urine output or plasma sample (i.e., irrespective of concentration or volume) can also be assayed, if desired.

The presence or concentration of vitamin C can be assayed using any suitable technique, i.e., a sensitive assay for vitamin C. A suitable limit of detection for such assay can be 0.01 mg/total urine volume (or about 0.01 mg/total urine volume), or 50 nmol/L (or about 50 nmol/L), for example, or even lower (e.g., 40 nmol/L (or about 40 nmol/L), about 30 nm/L (or about 30 nmol/L), 20 nm/L (or about 20 nmol/L), or about 10 nm/L (or about 10 nmol/L)). A preferred assay for the presence or concentration of vitamin C employs liquid chromatography, such as coulometric electrochemical detection with high performance liquid chromatography (HPLC) or liquid chromatography mass spectrometry (LCMS). Other methods for assaying for the presence or concentration of vitamin C can be used in the context of the present invention as well, preferably such methods that are at least as sensitive in vitamin C detection as HPLC with coulometric electrochemical detection or LCMS.

However assayed, the presence of vitamin C renal leak in the human subject is determined by assessing whether the collected urine output contains vitamin C and whether the concentration of vitamin C in the plasma sample is below the Minimum Elimination Threshold (MET). Without wishing to be bound by theory, the Minimum Elimination Threshold (MET) represents a threshold for plasma vitamin C concentration above which some renal filtration of vitamin C, and therefore some presence of vitamin C in the collected urine output, is appropriate in healthy subjects, and therefore not indicative of pathology. However, when the plasma concentration of vitamin C is sufficiently low (below the MET), the presence of vitamin C in the urine is clinically inappropriate and represents a sign of renal impairment or risk thereof, such as from a disease, planned procedure, genetic predisposition, or otherwise.

MET is related to the renal threshold. Vitamin C renal threshold is defined as the plasma vitamin C concentration during a bioavailability study at which vitamin C first appears in the urine during a repletion phase or when urinary elimination stops during a depletion phase. Experimentally, as determined herein using a physiology-based pharmacokinetic model (PBPK), renal thresholds of 48.5 (5.2) $\mu$M for healthy male human subjects and 58.3 (7.5) $\mu$M for healthy female human subjects have been calculated (expressed as "value (standard deviation)") (see Example 1). Similarly, using a population curve-fitting (PCF) model revealed similar renal threshold values of 48.3 $\mu$M in healthy male human subjects and 60.4 $\mu$M in healthy female human subjects have been calculated. Accordingly, experimentally as determined in the experimental Examples set forth herein, MET has been determined to be 38.1 $\mu$M when the human subject is male and 43.2 $\mu$M when the human subject is female (see Figure 2D). However, for purposes of the present invention, the MET value used in performing the inventive method can be taken to be within a range of from about 1 to about 3 standard deviations below the renal threshold. Thus, for purposes of the present invention, when the human subject is male, the MET value used in performing the inventive method can be a value within a range of between about 32.5 $\mu$M and about 43.5 $\mu$M, such as between 32.9 $\mu$M and 43.3 $\mu$M. Similarly, when the human subject is female, the MET value used in performing the inventive method can be a value within a range of between about 35.5 $\mu$M and about 51.0 $\mu$M, such as between 35.8 $\mu$M and 50.8 $\mu$M. Preferably, the MET value used in performing the inventive method approximates the MET derived experimentally as reported in the Examples sections below and can be thus about 38.5 $\mu$M when the human subject is male and about 43.5 $\mu$M when the human subject is female. Of course, the MET value used in performing the inventive method can be as derived experimentally as reported in the Examples sections below: 38.1 $\mu$M (or about 38.1 $\mu$M) when the human subject is male and 43.2 $\mu$M (or about 43.2 $\mu$M) when the human subject is female.

In addition to assessing the presence of vitamin C renal leak, the result can be quantified by further assessing the fractional excretion of vitamin C (FeVC) in the human subject. FeVC is a measurement of the degree of urinary vitamin C excretion relative to plasma vitamin C concentration corrected by creatinine clearance.

The FeVC of the human subject can be assessed using the same plasma sample and urine output collected from the human subject as described herein for use in assessing vitamin C renal leak. Alternatively, fresh plasma and urine, also obtained and collected during the predefined timed interval, which begins at time 0, can be obtained as described herein.

To assess FeVC, the volume of the urine output collected during the predefined timed interval is assessed, as is the amount and/or concentration of vitamin C and creatinine in the collected urine output and the plasma sample. The amount and/or concentration of vitamin C can be assessed using a sensitive assay for vitamin C, e.g., using liquid chromatography, as described above, such as coulometric electrochemical detection with HPLC or LCMS. A suitable limit of detection for such assay can be as discussed above, such as about 0.01 mg/total urine volume, or about 50 nmol/L, for example, or even lower (e.g., about 40 nmol/L, about 30 nm/L, about 20 nm/L, or even about 10 nm/L). Other methods for assaying for the presence or concentration of vitamin C can be used in the context of the present invention as well, preferably such methods that are at least as sensitive in vitamin C detection as HPLC with coulometric electrochemical detection or LCMS.

The amount and/or concentration of creatinine in the plasma sample and urine output collected during the predefined timed interval, which begins at time 0, can be assessed using any suitable technique. Creatinine is typically measured by standard clinical laboratory testing as part of a basic metabolic panel, a renal panel, or a comprehensive metabolic panel. Creatinine can be measured by point of care devices, and standard clinical laboratory automated multi-analyzers, e.g., Roche COBAS and the like.

With the amount and/or concentration of vitamin C in the urine output collected during the predefined timed interval, volume of the urine output collected during the predefined timed interval, concentration of vitamin C in the plasma sample, and amount and/or concentration of creatinine in both the collected urine output and plasma samples assessed, the FeVC can be calculated as "A" according to the following equation:

$$A = \frac{\dfrac{[urine\ vitamin\ C\ concentration\ (\mu M)] * [urine\ volume\ (L)]}{[plasma\ vitamin\ C\ concentration\ (\mu M)]}}{\dfrac{[urine\ creatinine\ concentration\ (\mu M)] * [urine\ volume\ (L)]}{[plasma\ creatinine\ concentration\ (\mu M)]}}$$

It will be observed that the inventive assessment of vitamin C renal leak leads to several useful clinical and research uses. Disclosed but not claimed is a method of treating a human subject not exhibiting the presence of vitamin C renal leak ("vitamin C renal leak negative") with an intervention, procedure, or therapy bearing a risk of renal impairment. In this context, the method comprises assessing vitamin C renal leak in a human subject in need of treatment with an intervention, procedure, or therapy bearing a risk of renal impairment, or a candidate for such treatment. For such human subjects, when the assessment does not indicate the presence of vitamin C renal leak, the subject may be judged to be amenable to treatment with an intervention, procedure, or therapy bearing a risk of renal impairment. The method then can proceed with treating such a human subject with the intervention, procedure, or therapy bearing a risk of renal impairment. When used in this context, the assessment of vitamin C renal leak can be performed alone (as the only assessment of the human subject in connection with evaluating a human subject as a candidate for an intervention, procedure, or therapy bearing a risk of renal impairment), or the assessment of vitamin C renal leak can be used in conjunction with other clinical tests and parameters, such as are known to those of ordinary skill in the art to be useful for clinical risk assessment.

In this context, an intervention, procedure, or therapy bearing a risk of renal impairment can comprise a procedure such as renal or vascular surgery (e.g., coronary artery bypass surgery, renal transplant surgeries, and the like) or surgery involving the risk or occurrence of acute kidney injury (for example (but not limited to) the occurrence of hypotension or acute blood loss, among others).

The intervention, procedure, or therapy that bears a risk of renal impairment also can comprise an imaging procedure involving the use of radiocontrast media that can pose a risk of injury to the kidneys (e.g., CT contrast procedures, MR contrast procedures, or other similar imaging procedure as well as contrast imaging-guided procedures such as arteriography and venography). Examples of radiocontrast media that can pose a risk of injury to the kidneys include, but are not limited to, Iodinated/iodine-based contrast media such as iohexol (OMNIPAQUE 350), iopamidol (ISOVUE 370), metrizoate (ISOPAQLTE 370), and the like, Gadolinium based contrast agents, and other potentially nephrotoxic contrast agents.

The inventive method also can be employed in the context of an intervention, procedure, or therapy that bears a risk of renal impairment due to the use of anesthesia agents with a surgical procedure where there is risk of hypotension (for example (but not limited to) procedures involving patients with chronic spinal cord dysfunction, patients with spinal cord injury, recipients of epidural anesthesia where there is potential risk of hypotension, patients experiencing acute blood loss, and the like).

An intervention, procedure, or therapy bearing a risk of renal impairment, in the context of the present invention, also can comprise the exposure to or administration of a nephrotoxic agent to the human subject. Such an agent can be a pharmaceutically effective agent (e.g., a drug substance) or other chemical having clinical utility, but which poses a risk of nephrotoxicity, such as contrast media as discussed herein. Other example of such nephrotoxic agents comprise, but are by no means limited to, chemotherapy drugs such as but not limited to alkylating agents (e.g., cyclophosphamide, melphalan, and the like); antimetabolites (e.g., gemcitabine, hydroxyurea, methotrexate, and the like); anti-microbial agents (e.g., Taxanes, vinca alkaloids, and the like); anti-tumor antibiotics (e.g., bleomycin, etc.); platinum compounds (e.g., cisplatin, carboplatin, and the like); compounds such as arsenic trioxide, irinotecan, and the like; immunomodulatory agents (e.g., thalidomide, lenalidomide, and the like); proteosome inhibitors (e.g., bortezomib, carfilzomib, and the like); immune checkpoint inhibitors (i.e. pembrolizumab); and other chemotherapy drugs. Other non-limiting examples include antibiotics such as Gentamycin, Tobramycin, Amphotericin B, and the like; anti-inflammatory agents, such as nonsteroidal anti-inflammatory drugs (e.g., ketorolac, ibuprofen, naproxen, etc.); anti-viral agents, such as drugs used to treat HIV (e.g., TENOFOVIR) and hepatitis C (e.g., RIBAVIRIN), among others. In the context of the present invention, a nephrotoxic agent also can be one whose nephrotoxicity may depend on the condition of a patient or other human subject; in this respect, for example, hydroxyurea exhibits nephrotoxicity when used in human subjects with sickle cell disease.

In another context, assessment of vitamin C renal leak in a human subject in accordance with the inventive method can lead to the identifying a general clinical risk of renal impairment to a human subject. In this context, the invention provides a method of identifying a clinical risk of renal impairment to a human subject comprising assessing vitamin C renal leak in a human subject, whereby an assessment that indicates the presence of vitamin C renal leak in the human subject ("vitamin C renal leak positive") identifies a present or future clinical risk of renal impairment to the human subject. Such a risk of renal impairment can be due to one or a combination of clinical factors not manifested in the human subject at the time of the assessment of the presence of vitamin C renal leak, whereby the method can be used to identify future clinical risk to such human subjects. Such identification of the risk of renal impairment can be particularly useful for human subjects not exhibiting renal impairment at the time of the assessment of the presence of vitamin C renal leak (e.g., human subjects not exhibiting albuminuria and/or proteinuria; however, as noted above, the invention is not limited in its use to such human subjects. A vitamin C renal leak positive assessment can serve to inform such human subjects and their clinicians of a future risk to their kidneys due to one or a combination of factors, which can assist in their adopting dietary and other habits before the onset of disease or the occurrence of damage from disease or administering prophylaxis, and thus can help reduce the risk of future renal impairment and otherwise aid in clinical assessment, treatment, and management of such human subjects.

In many such applications of the method, particularly for early assessment of clinical risk of renal impairment, the assessment of vitamin C renal leak can be performed alone (as the only assessment of the human subject in connection with identifying a general clinical risk of present or future renal impairment). However, it will be apparent that the inventive method for assessing vitamin C renal leak can be used in conjunction with other clinical tests and observations, as well as relevant factors of a human subject's medical history, family history, work history and the like, such as are known to those of ordinary skill in the art to be useful for clinical risk assessment. For example, in some preferred embodiments, the inventive method of identifying a clinical risk of renal impairment to a human subject generally through a positive assessment of vitamin C renal leak can, for example, assist in the clinical assessment, treatment, and management of human subjects with a family history of diseases involving renal impairment (for example, a family history of diabetes, cardiovascular disease, autoimmune diseases such as lupus, or other relevant family medical history).

The inventive method of identifying a clinical risk of renal impairment to a human subject generally through a vitamin C renal leak positive assessment also can assist in the clinical assessment, treatment, and management of such human subjects who have genetic predispositions to develop renal impairment (such as, for example, human subjects bearing the genetic abnormality associated with Fabry Disease, Autosomal Dominant Polycystic Kidney Disease, Tuberous Sclerosis, Alport Syndrome, Von-Hippel Lindau Syndrome, and the like). Similarly, a positive assessment of vitamin C renal leak can aid in identifying a clinical risk of renal impairment to a human subject having increased risk based on medical history (including, but not limited to, a disease), and thereby can be beneficial in the clinical assessment, treatment, and management of such human subjects. The inventive method of identifying a clinical risk of renal impairment to a human subject generally through a vitamin C renal leak positive assessment also can assist in the clinical assessment, treatment, and management of human subjects bearing other risk factors, such as a history of smoking, drug use, or a work history (such as, but not limited to, occupational environmental exposures to potentially nephrotoxic chemicals or solvents in agricultural, furniture-making, and other industries) that presents a risk of renal damage. For example, some human subjects on heroin can develop IV nephropathy, and the inventive method can assist in identifying an increased clinical risk of renal impairment for such human subjects who are assessed to be vitamin C renal leak positive.

The inventive method of identifying a clinical risk of renal impairment to a human subject generally through a positive assessment of vitamin C renal leak also can assist in the clinical assessment, treatment, prophylaxis, and management of human subjects who might require or benefit from an intervention (including prophylaxis), procedure, or therapy bearing a risk of renal impairment, despite their status as having a positive assessment of vitamin C renal leak. Examples of such human subjects include those who are undergoing or are candidates for a course of therapy or prophylaxis or clinical observation that would benefit from administration of a nephrotoxic agent, for example patients who are candidates for imaging procedures involving potentially nephrotoxic contrast agents and medications, such as those discussed herein, that can pose a risk of injury to the kidneys. Knowledge that a given human subject who is a candidate for such a procedure or administration of a nephrotoxic agent and who also has an assessment of being vitamin C renal leak positive may assist in monitoring such human subject before, during, and after the procedure or administration of a nephrotoxic agent to identify signs of renal damage or impairment or lead to enhanced vigilance for the possibility of complications from such procedure or administration of a nephrotoxic agent and to more promptly assess, treat, and manage such complications, should they arise.

The inventive method of identifying a clinical risk of renal impairment to a human subject generally through a positive assessment of vitamin C renal leak also can assist in the clinical assessment, treatment, prophylaxis, and management of human subjects in instances in which the clinical risk of renal impairment is due to a disease. In this context, assessment of vitamin C renal leak in a human subject in accordance with the inventive method can be used for screening for the clinical risk of a disease in the human subject. In accordance with this aspect, the invention provides a method of identifying or screening for a clinical risk of a disease in a human subject comprising assessing vitamin C renal leak in the human subject as discussed above, whereby an assessment that indicates the presence of vitamin C renal leak in the human subject (a vitamin C renal leak positive result) identifies a clinical risk of a disease in the human subject. Thus, a vitamin C renal leak positive result supports a conclusion that the human subject is at clinical risk of a disease. A Vitamin C renal leak positive assessment similarly can be used as the basis for diagnosing a disease in a human subject. In this context, the invention provides a method of diagnosing a disease in a human subject comprising assessing vitamin C renal leak in a human subject as discussed above, whereby an assessment that indicates the presence of vitamin C renal leak in the human subject (a vitamin C renal leak positive result) supports a diagnosis of a disease in the human subject.

Such identification of and screening for the clinical risk of a disease, or diagnosis of disease, can be particularly useful for human subjects not exhibiting renal impairment or other signs of disease at the time of the assessment of the presence of vitamin C renal leak (e.g., human subjects not exhibiting albuminuria and/or proteinuria); however, as noted above, the invention is not limited in its use to such human subjects. The inventive method can be used in the context of a variety of diseases and is particularly useful in the context of diseases presenting with complications or progressions comprising renal impairment. For such human subjects, a vitamin C renal leak positive assessment can serve to identify or screen for clinical risks of disease and to diagnose such diseases earlier than currently possible and before such diseases progress to the point of causing damage that leads to albuminuria, proteinuria, or other symptoms or damage. Early assessment of the risk of disease in such human subjects, and/or early diagnosis, can result in enhanced clinical outcomes due to earlier intervention, procedure, management, or therapy as prophylaxis against or therapeutic treatment of the disease. Disclosed but not claimed is a method which then can proceed with treating such a human subject with the intervention, procedure, management, or therapy as prophylaxis against or therapeutic treatment of the disease. While in many such applications, particularly for early assessment of risk and/or diagnosis, the assessment of vitamin C renal leak can be performed alone (as the only assessment of the human subject in connection with identification of and screening for the clinical risk of a disease, or diagnosis of disease), in other applications, the assessment of vitamin C renal leak can be used in conjunction with other clinical tests, such as are known to those of ordinary skill in the art to be useful for clinical risk assessment or diagnosis of disease.

In another context, the invention provides a method of monitoring disease state in a human subject. In this context, the method comprises obtaining at least two assessments of renal leak in the human subject, which are compared with each other. Thus, the method comprises performing a first assessment of vitamin C renal leak in the human subject and later performing a second assessment of vitamin C renal leak in the human subject, both using the method described herein for assessing vitamin C renal leak. Following the at least two assessments of vitamin C renal leak in the human subject, the results of the assessments are compared. Thus, the first assessment of renal vitamin C leak is compared with the second assessment of renal vitamin C leak, and if there is a third or fourth (or more) iteration of the method, the results of such assessments also can be compared.

It will be observed that, relative to each other, when a first assessment of vitamin C renal leak does not indicate the presence of renal vitamin C leak in the human subject and a second assessment of vitamin C renal leak indicates the presence of vitamin C renal leak in the human subject, a determination of disease progression in the human subject between the two assessments of vitamin C renal leak can be made. Conversely, relative to each other, when the first assessment of vitamin C renal leak indicates the presence of renal vitamin C leak in the human subject and the second assessment of vitamin C renal leak does not indicate the presence of vitamin C renal leak in the human subject, a determination of disease regression in the human subject between the two assessments of vitamin C renal leak can be made.

Furthermore, in instances in which both a first assessment and a second assessment of vitamin C renal leak in the human subject are positive (i.e., the first assessment of vitamin C renal leak indicates the presence of renal vitamin C leak in the human subject and the second assessment of vitamin C renal leak also indicates the presence of renal vitamin C leak in the human subject) or in instances in which both a first assessment and a second assessment of vitamin C renal leak in the human subject are negative (i.e., the first assessment of vitamin C renal leak does not indicate the presence of renal vitamin C leak in the human subject and the second assessment of vitamin C renal leak also does not indicate the presence of renal vitamin C leak in the human subject), a determination of nether disease progression or disease regression between the two assessments of vitamin C renal leak is made. Rather, in such instances, a determination of disease stasis can be made.

In application, the inventive method of monitoring disease state in a human subject is particularly useful for a human subject who either has a disease and is undergoing treatment of the disease or for a human subject who is receiving prophylaxis against the development of the disease. In connection with the therapeutic treatment of such a human subject, as the therapeutic treatment of the disease is administered to the human subject, the method is employed to assess the response of the human subject to such therapeutic treatment (e.g., by determining the regression or stasis of the disease in response to the therapeutic treatment). Similarly, in connection with the prophylactic treatment of such a human subject, as the prophylaxis administered to the human subject, the method is employed to assess the response of the human subject receiving prophylaxis against the development of the disease (e.g., by determining the progression or development of the disease despite prophylaxis or stasis in response to the prophylaxis). While in some applications, the at least two assessments of vitamin C renal leak can be performed alone (as the only assessments of the human subject in connection with monitoring disease state in a human subject), in other applications, the assessment of vitamin C renal leak can be used in conjunction with other clinical tests, observations, or parameters (e.g., history) useful for monitoring disease state in the human subject, such as are known to those of ordinary skill in the art.

In another context, the invention has predictive value in the context of disease treatment or prophylaxis. In this context, the invention provides method of predicting the response of a human subject to therapeutic treatment or prophylaxis. In accordance with this aspect of the invention, the human subject can be a patient in need of therapeutic treatment or a human subject in need of prophylaxis against the development of the disease (e.g., a human subject at risk of developing the disease). In this context, the method comprises assessing vitamin C renal leak in the human subject as discussed above, whereby an assessment that indicates the presence of vitamin C renal leak in the human subject (a vitamin C renal leak positive result), results in a prediction that the human subject will be responsive to the therapeutic treatment or prophylaxis. In certain applications, the assessment of vitamin C renal leak can be performed alone (as the only assessment of the human subject in connection with predicting the response of a human subject to therapeutic treatment or prophylaxis), while in other applications, the assessment of vitamin C renal leak can be used in conjunction with other clinical tests, observations, or parameters (e.g., history), such as are known to those of ordinary skill in the art to have predictive value. The method can then proceed with administering to the human subject having the vitamin C renal leak positive result the therapeutic treatment or prophylaxis, which can be, for example, an intervention, procedure, or therapy (e.g., treatment with a pharmaceutical agent) as therapeutic treatment of or prophylaxis against the development of the disease.

In another context, which is disclosed but not claimed, a method of administering a therapeutic treatment or prophylaxis to a human subject exhibiting the presence of vitamin C renal leak is provided. The human subject can be a patient in need of therapeutic treatment or a human subject in need of prophylaxis against the development of the disease (e.g., a human subject at risk of developing the disease). In this context, the method comprises assessing vitamin C renal leak in the human subject as discussed above. In certain applications, the assessment of vitamin C renal leak can be performed alone (as the only assessment of the human subject in connection with administering a therapeutic treatment or prophylaxis to the human subject), while in other applications, the assessment of vitamin C renal leak can be used in conjunction with other clinical tests, observations, or parameters (e.g., history), such as are known to those of ordinary skill in the art. If the assessment indicates the presence of vitamin C renal leak in the human subject (a vitamin C renal leak positive result), the method then proceeds with administering to the human subject the therapeutic treatment or prophylaxis, which can be, for example, an intervention, procedure, or therapy (e.g., treatment with a pharmaceutical agent) as therapeutic treatment of or prophylaxis against the development of the disease.

Disclosed but not claimed is that a human subject (which can be a patient having a disease in need of therapeutic treatment thereof or a non-diseased human subject in need of prophylaxis against the development of a disease) is administered therapeutic or prophylactic treatment. Such treatment can comprise, for example, any suitable intervention, procedure, or therapy as therapeutic treatment of or prophylaxis against the development of a disease. As noted above, such a treatment can be or comprise a procedure (e.g., surgical or imaging) or agent bearing a risk of renal impairment, but the invention is not limited to the use of potentially nephrotoxic agents or other treatments bearing a risk of renal impairment. Persons of ordinary skill in the art are able to select an appropriate intervention, procedure, or therapy as therapeutic treatment of or prophylaxis. In many applications, the therapeutic treatment of or prophylaxis used comprises a pharmaceutical agent, i.e., a drug substance. The dosages and routes of administration of such agents are known to those of ordinary skill in the art, and such agents can be administered to patients or other human subjects in accordance with such known methodology.

For example, particularly useful in the treatment of and prophylaxis against diabetes (e.g., type 1 or type 2 diabetes) and possibly retinopathy, the treatment can comprise the administration of an angiotensin converting enzyme (ACE) inhibitor and/or an aldosterone receptor blocker (ARB). Suitable ACE inhibitors include, but are not limited to, LISINOPRIL, ENALAPRIL, RAMIPRIL, and the like. Suitable ARB blockers include, but are not limited to, LOSARTAN, VALSARTAN, OLMESARTAN, and the like. In other embodiments, the treatment of diabetes can comprise the administration of an antihyperglycemic agent, such as insulin, or oral hypoglycemic agents such as METFORMIN, GLIPIZIDE, and the like. Other suitable anti-diabetic medications and treatments can be used as well.

In embodiments particularly relevant to cardiovascular diseases, the treatment can comprise, for example, the administration of a GLP-1 agonist, such as SEMAGLUTIDE, LIRAGLUTIDE, DULAGLUTIDE, and the like. In other embodiments, the treatment can comprise the administration of an SGLT-2 inhibitor, such as CANAGLIFLOZIN, DAPAGLIFLOZIN, EMPAGLIFLOZIN, and the like. Other suitable medications and treatments used in the context of cardiovascular diseases can be used as well.

For embodiments involving the management (treatment of or prophylaxis against) of Fabry disease, a particularly useful approach comprises enzyme replacement therapy, which comprises the administration of Recombinant alpha-galactosidase A (alpha-Gal A) to the human subject having Fabry disease or the genetic abnormality leading to its development.

In other embodiments, the relevant disease can comprise any etiology involving the kidney, for example an acute, chronic, or acute-on-chronic renal disease. Examples of acute renal diseases include, but are not limited to, those with pre-renal etiologies (e.g., dehydration and, sepsis), those with renal etiologies. (e.g., acute tubular necrosis and acute interstitial nephritis), or post-renal etiologies (e.g., urolithiasis; benign prostate hyperplasia, urinary tract outlet obstruction of ureters, bladders, and the like). Examples of chronic renal diseases include, but are not limited to, diabetic kidney disease, hypertensive kidney disease, and the like. An example of an acute-on-chronic renal disease includes sepsis in the setting of chronic kidney disease, but other similar acute-on-chronic conditions can arise.

As noted, the inventive method involving assessment of vitamin C renal leak is useful in the context of a wide variety of diseases and clinical conditions. As but one example, such a disease or condition can comprise an etiology involving the kidney. For example, a disease comprising etiology involving the kidney can comprise an allergic or atopic reaction (such as, but not limited to, acute interstitial nephritis, drug induced allergic reactions, and the like). As another example, a disease comprising etiology involving the kidney can comprise an autoimmune, inflammatory, or rheumatological disorder, such as (but not limited to) systemic lupus erythematosus, glomerulonephritis, and the like. As another example, a disease comprising etiology involving the kidney can comprise vasculitis, such as (but not limited to) granulomatosis with polyangiitis, microscopic polyangiitis, and the like. As another example, a disease comprising etiology involving the kidney can comprise an infection, such as (but not limited to) sepsis secondary to viruses, bacterial, fungal, UTI, HIV nephropathy, Hepatitis C nephropathy, and the like. Other examples of diseases comprising etiology involving the kidney also include, but are not limited to, metabolic disorders having renal implications, such as diabetes, obesity, metabolic syndrome, hypothyroidism, Fabry disease, etc. Other examples of diseases impacting the kidney include certain neoplastic disorders, such as malignancies (e.g., involving the blood or kidney), multiple myeloma, and the like. Other examples of diseases comprising etiology involving the kidney include neurological conditions (such as, but not limited to, altered mental state from uremia, renal failure, and the like), structural disorders (such as, but not limited to, renal malformations, post-renal obstruction, kidney/urological stones, and the like), and vascular disorders (such as, but not limited to, renovascular HTN, cardio-renal syndrome, and the like). The treatment or prophylaxis of such renal diseases and diseases with renal etiologies in the context of the present invention can comprise the administration of suitable pharmaceutical agents known to those of ordinary skill to be safe and effective for such purposes. The dosages and routes of administration of such agents also are known to those of ordinary skill in the art, and such agents can be administered to patients or other human subjects in the context of the present invention in accordance with such known methodology.

As noted, the inventive method for assessing vitamin C renal leak can be used in the contact of a variety of other diseases or conditions, including but not limited to autoimmune diseases such as lupus or those involving the kidneys, diabetic nephropathy, chronic urinary tract infections, Fabry disease, retinopathy, cardiovascular disease, heart failure, stroke, a cancer (such as a cancer involving the blood or kidney cancer), or HIV disease. The treatment or prophylaxis of such diseases and conditions in the context of the present invention can comprise the administration of suitable pharmaceutical agents known to those of ordinary skill to be safe and effective for such purposes. The dosages and routes of administration of such agents also are known to those of ordinary skill in the art, and such agents can be administered to patients or other human subjects in the context of the present invention in accordance with such known methodology.

Given that the present invention is predicated on a method of assessing vitamin C renal leak, in many instances, the treatment or prophylaxis that can be employed as clinical treatment or prophylaxis of disease herein can comprise the administration of vitamin C. In this context, the vitamin C can be administered via a given human subject's dietary intake of food or oral supplements (e.g., multivitamin supplements) or, particularly in a clinical or hospital setting, intravenously at a suitable dosage. In this sense, vitamin C renal leak status has novel importance for vitamin C dietary recommendations and guidelines. Current recommendations are based on vitamin C pharmacokinetics studies and provide guidelines for healthy people. Increased vitamin C intake is suggested for pregnant women, because of estimated requirements of the fetus, and for smokers, because of estimated increased loss from oxidants in cigarette smoke. However, there are no current guidelines for increased intake in chronic diseases. Based on evidence provided herein, it is possible that vitamin C renal leak may lead to vitamin C losses that should be replaced by higher recommended intake in people with diabetes (e.g., type 1 or type 2 diabetes) and other conditions discussed herein.

EXAMPLES

The following experimental Examples further illustrate the invention but, of course, should not be construed as in any way limiting its scope.

In these Examples, vitamin C renal leak criteria were tested and evaluated in separate clinical studies involving more than five different cohorts of healthy controls or human subjects with chronic disease. Findings revealed significant associations between vitamin C renal leak status and clinical variables involving renal function and blood glucose (glycemic control), among others.

EXAMPLE 1

This example demonstrates the presence of vitamin C renal leak in human subjects with Fabry Disease. A renal threshold for vitamin C determined in healthy individuals was disrupted in men with Fabry Disease.

Briefly, to determine renal threshold, the plasma concentration at which vitamin C first appears in urine, 17 healthy subjects underwent depletion-repletion studies, with 10-14 pharmacokinetics studies per subject with vitamin C dose range of 15-1250 mg daily. Renal threshold was estimated using physiology-based pharmacokinetics modeling. Applying renal threshold 95% confidence intervals, the plasma concentration below which no vitamin C was expected in urine of healthy people: a minimal elimination threshold, was estimated. Renal leak was defined as presence of vitamin C in urine with plasma concentrations below the minimal elimination threshold. Criteria was tested in a cohort of men with Fabry disease (33) and healthy controls (34) using matched plasma and urine samples to determine renal leak prevalence, fractional excretion of vitamin C and associated clinical parameters. Mice knockout models were used for proof-of-concept studies. Vitamin C was measured using coulometric electrochemical detection with high performance liquid chromatography.

Vitamin C renal threshold in healthy men and women was 48.5$\mu$M(5.2) and 58.3$\mu$M(7.5) respectively. Compared with healthy men, Fabry cohort had 16-fold higher odds of renal leak (6% v 52%:OR16.3, p<0.001) and higher fractional excretion of vitamin C (p<0.001). Renal leak predictive clinical variables were glomerular filtration rate (p< 0.001); protein creatinine ratio (p< 0.001) and protein excretion (p<0.001). Renal threshold and its disruption were recapitulated in mice.

The studies in this Example had three major objectives. First, in healthy men and women normal vitamin C renal thresholds was characterized using data obtained from vitamin C depletion-repletion and pharmacokinetics studies. Second, renal threshold data was utilized to determine when urinary vitamin C excretion may be inappropriate for a given plasma vitamin C concentration. Inappropriate urinary vitamin C excretion can be considered a renal leak. Third, these criteria were utilized for renal leak to investigate vitamin C pathophysiology in a disease population. It was hypothesized that a renal threshold for vitamin C might be dysregulated in a disease that affects renal tubules, and the consequences would be inappropriate urinary vitamin C excretion (renal leak). To test this hypothesis, urinary vitamin C excretion and prevalence of renal leak in men with Fabry disease and healthy male controls was studied.

Abbreviations used in the Example include: BMI, body mass index; PCR, protein creatinine ratio; ACE, angiotensin-converting enzyme inhibitor; ARB, angiotensin receptor blocker; AST, aspartate aminotransferase; ALT, alanine aminotransferase; FeVC: Fractional Excretion of Vitamin C; MET: Minimum Elimination Threshold; PBPK: Physiology-Based Pharmacokinetics model; and PCF: Population Curve Fitting model.

## METHODS

### Determination of vitamin C renal threshold and renal leak criteria: depletion-repletion studies.

*Study design, settings:* This was a nonrandomized interventional study aimed at determining normal vitamin C renal threshold in healthy men and women using a vitamin C depletion-repletion protocol (DK-92-0033). Pharmacokinetics components of depletion-repletion study design have been described previously (see supplementary methods). The protocol was approved by the Institutional Review Board, National Institutes of Diabetes and Digestive and Kidney diseases (NIDDK), National Institutes of Health (NIH).

*Participants, Interventions:* Healthy men and women aged 18 to 35 years without any acute or chronic medical conditions. Details on eligibility and study interventions are described in supplementary methods.

*Study outcomes, analyses:* The major study outcomes were to determine normal vitamin C renal threshold (the vitamin C plasma concentration in which filtered vitamin C first appears in in urine), minimal elimination threshold [MET] (the plasma vitamin C concentration 2 standard deviations below the renal threshold) and to determine criteria for inappropriate urinary vitamin C excretion (renal leak). Full details regarding studying outcomes and analyses are provided in supplementary methods. Briefly, vitamin C renal threshold is estimated as the plasma vitamin C concentration during a bioavailability study at which vitamin C first appears in the urine during a repletion phase or when urinary elimination stops during a depletion phase. Each subject had 5-7 oral and intravenous pharmacokinetic studies over a 13-fold or greater dose range. Data from the NIH depletion study in men and women were used to estimate the vitamin C renal threshold by two different methods: physiology-based pharmacokinetics modeling (PBPK), and population curve fitting with empirical functions (PCF). PBPK utilized a physiological-based pharmacokinetics model of vitamin C that incorporated nonlinear absorption, tissue distribution, and renal elimination/reabsorption kinetics with 5 interconnected compartments (absorption; central; tissue; renal tubule; urine) as well as gut loss and tissue metabolism. Based on data from healthy men, this model facilitated description of pharmacokinetics-time profiles of vitamin C in plasma, urine, and tissues simultaneously. Goodness of fit analyses support the use of this model to data from healthy women. For population curve fitting (PCF), plasma and urine values were analyzed using nonlinear mixed-effect modeling. PCF fits empirical functions to data from all subjects of each sex to estimate the renal threshold for that population of men and women. Using PBPK analyses, minimal elimination threshold (MET) was defined as that plasma concentration 2 standard deviations below the mean renal threshold for men and for women based on guidelines for estimating dietary reference intakes. When plasma vitamin C concentrations are less than the MET, urinary vitamin C excretion is inappropriate, a term referred to as renal leak. (see supplementary methods).

**Vitamin C renal leak in Fabry's disease and Healthy controls.**

*Study design:* This was a non-randomized cross-sectional cohort study aimed at investigating the prevalence and clinical characteristics of *vitamin* C renal leak in men with Fabry disease and healthy male controls. The protocols were approved by the Institutional Review Boards of NINDS and NIDDK, NIH.

*Participants:* Men with Fabry disease who were actively participating in NIH protocols evaluating the open label use of enzyme replacement therapy (ERT) in Fabry disease (95-N-0121, 00-N-0185 and 02-N-0220), *ClinicalTrials*.gov Identifiers: NCT00001491 and NCT00068107) were recruited. The male predominance in Fabry disease is based on the X-linked inheritance pattern. Accordingly, healthy men aged 18-58 were recruited on a rolling basis as controls for the Fabry cohort, under NIH protocol 04-DK-0021 (arm 3). Exclusion criteria included acute or chronic illness, alcohol abuse or tobacco use. Exclusion criteria were chosen to avoid confounding study findings, given reported association of these conditions with vitamin C dysregulation.

*Interventions:*

*Pre-study vitamin C dietary restriction in healthy controls:* Men with Fabry's disease have comparatively lower plasma vitamin C concentrations. Healthy controls were instructed to adhere to a low-vitamin C diet 3 weeks prior to the inpatient study to ensure that vitamin C plasma concentrations were matched in both groups and to avoid biased comparison of renal leak prevalence.

*Matched urine and plasma vitamin C measurements:* Following an overnight fast, on the morning of study, subjects were instructed to empty their bladder. After a 1-hour interval, a spot urine sample (termed 1h urine) and blood samples were obtained for measurement of vitamin C concentrations, chemistry, and related studies. Following the initial spot urine collection, subjects were hospitalized for another 24 hours. They consumed a hospital diet free of supplements or foods items with no or low vitamin C content. On morning of day 2, collection of all voided urine for 24 hours (termed 24h urine) was obtained and subjects were discharged home.

*Study outcomes:*

Primary endpoint was the prevalence of vitamin C renal leak in the Fabry's cohort compared with healthy controls. Renal leak status (leak vs no leak) was determined using criteria described in depletion-repletion studies above. While useful for estimating epidemiological questions such as prevalence, a limitation of the categorical binary criteria is the inability to quantify the magnitude of urinary vitamin C excretion relative to plasma concentrations (i.e., the degree of renal leak). To address this limitation, renal leak was also expressed as fractional excretion of vitamin C (FeVC) defined as follows:

$$A = \frac{\dfrac{[urine\ vitamin\ C\ (mg)] * [urine\ volume\ (L)]}{[plasma\ vitamin\ C\ concentration\ (\mu M)]}}{\dfrac{[urine\ creatinine\ (mg)] * [urine\ volume\ (L)]}{[plasma\ creatinine\ concentration\ (\mu M)]}}$$

The secondary endpoint was median difference in FeVC in Fabry disease cohort compared with healthy controls. The other exploratory endpoint was evaluating the clinical variables associated with vitamin C renal leak expressed as FeVC (see Table 1, which concerns baseline characteristics of participants in the vitamin C depletion-repletion and vitamin C renal leak study).

*Sample collection and vitamin C assay.*

Urine and plasma vitamin C were analyzed by HPLC with coulometric electrochemical detection as described, limit of detection 0.01mg/total urine volume (see supplementary methods). Other blood and urine chemistry measurements were performed, using standard laboratory methods, by the department of laboratory medicine Clinical Laboratory Improvement Amendments CLIA ID Number 21D0665373), Clinical Research Center, National Institutes of Health

*Statistical Analysis*

Data were presented within groups as counts(%) for discrete-valued variables, and as median (interquartile range, IQR) or mean (standard deviation, SD) for continuous-valued variable per the distribution of each unless otherwise indicated in the table or figure descriptions. Group-specific estimates and comparative measures (e.g., odds ratios) were accompanied by their corresponding 95% confidence intervals and p-values unless otherwise indicated. Plotted estimates lacking visible confidence intervals meant the confidence intervals' widths were smaller than the symbol sizes on the requisite scale. In the case of estimated proportions, an exact binomial confidence intervals designed to match a 'central' Fisher's Exact Test were employed. In the case of estimated quantiles such as medians, a quantile regression approach yielded confidence intervals for group differences; in the case of the zero-inflated and highly skewed fractional excretion measure of renal leak (which lacked a Box-Cox power transformation parameter estimate consistent within sampling variability across timepoints; see supplemental methods) a Wilcoxon-Mann-Whitney test of location shift between groups was further employed in order to corroborate findings from median differences (with Hodges-Lehmann estimates of shift reported as in Figure 3F). When screening multiple variables for association with renal leak measures (e.g., Table 3), multiplicity adjustments were conducted using the Hommel procedure to account for positive correlations among associated p-values. All analyses were conducted, and figures produced using R v3.6.2+ (with the packages exactci, exact2x2, forestplot, ggplot2, multxpert, quantreg, MASS).

## SUPPLEMENTARY METHODS

### Determination of vitamin C renal threshold using depletion-repletion studies

*Interventions*

Vitamin C renal threshold is defined as the plasma vitamin C concentration at which vitamin C appears in the urine or when urinary elimination stops. Because urine samples collected at variable intervals were not synchronized with plasma samples, the time at which vitamin C first appears in the urine or when vitamin C urinary elimination became zero could not be determined from raw data alone. Mean plasma vitamin C concentration at the midpoint of each urine collection was calculated by interpolation of measured plasma vitamin C concentrations. Vitamin C exhibits saturable and/or nonlinear absorption, tissue distribution, elimination, and re-absorption kinetics, making calculation of renal threshold by interpolation alone inaccurate. Since the rate of vitamin C loss from tubules to bladder is high and the lowest limit of detection (LLD) of vitamin C assay used in this study is extremely low (about 5 nmol/L), the value of $C_{tube,t}$ (Vitamin C concentration in the tubule at time t) is approximately the same as $C_{urine,t}$ (Vitamin C concentration in urine at time t). Substituting the individual PK parameters and using the lowest limit of detection (LLD) for $C_{tube,t}$, $C_{plasma,t}$ (Concentration of vitamin C in plasma at time t) for each individual can be estimated (Table 2). Using the data from the NIH depletion repletion study, vitamin C renal threshold was estimated by two different methods: Physiology-Based Pharmacokinetics model (PBPK) and population curve fitting (PCF).

*Physiology based Pharmacokinetics model (PBPK):*

The previously published pharmacokinetic model of vitamin C incorporated nonlinear absorption, tissue distribution and renal elimination/reabsorption kinetics with five connected compartments (depot, central, tissue, kidney, and urine) to describe the pharmacokinetics-time profiles of vitamin C in plasma, urine and tissue simultaneously, based on data from 7 men. Goodness of fit analysis support the use of the pharmacokinetics model to data collected from 15 normal women. Pharmacokinetics parameters for vitamin C were estimated and were used to simulate renal tubular vitamin C concentration profile. When the amount of filtered vitamin C exceeds renal tubular reabsorptive capacity, vitamin C appears in the urine. The point at which renal threshold is reached (when filtration and reabsorption are in equilibrium) can be represented as

$$\frac{dC_p}{dT} = (Rate\ of\ elimination) - (Rate\ of\ reabsorption) \quad (1)$$

$$\frac{dC_p}{dT} = K_e \times C_{plasma,t} - \frac{K_{(re-abs)} \times C_{tube,t}}{K_{re50} + C_{tube,t}} = 0 \quad (2)$$

With $dC_p/dT$, Rate of plasma vitamin C concentration change at time t; Ke, Elimination constant; $C_{plasma,t}$, plasma vitamin C concentration at time t, $K_{(re-abs)}$, Reabsorption constant; $C_{tube,t}$, Vitamin C concentration in the tubule at time t, and $K_{re50}$, Concentration in the tubule at which reabsorption rate reaches 50% of maximum reabsorption rate.

The $C_{plasma}$ that fulfill the above equation is the plasma concentration threshold (renal threshold). At which time, the rate of vitamin C dispense into the tubule equals zero. The equation 2 can be rearranged as

$$C_{plasma,t} = \frac{\frac{K_{(re-abs)} \times C_{tube,t}}{K_{re50} + C_{tube,t}}}{K_e} \qquad (3)$$

Since the rate of vitamin C from tubules to bladder is high and the lowest limit of detection of vitamin C assay extremely low (about 50 nmol/L), the value of vitamin C concentration in the tubule at time t ($C_{tube,t}$) is approximately same as the vitamin C concentration in urine at time t ($C_{urine,t}$). By substituting the individual PK parameters obtained via the physiological-based pharmacokinetic modeling analysis with each individual's data and using the LLD for vitamin C concentration in the tubule at time t ($C_{tube,t}$), the vitamin C plasma concentration at time t ($C_{plasma,t}$) for each individual was estimated (Table 2).

*Population Curve Fitting (PCF):*

Different non-parametric empirical functions were explored to best describe the combined urine data versus plasma vitamin C value of all subjects. After the structure function (model) was selected using goodness-of-fit assessments, plasma and urine data analyses were finalized using Nonlinear Mixed-Effect (MLME) modeling for population fit with S-Plus for Windows (version 8, Insightful Corp. Seattle, WA) to estimate the parameters account for inter-subject variabilities.

A Michealis-Menten equation (equation 4) was selected and fitted to data to estimate the parameters Vm and $K_M$ (maximum rate and Michaelis constant, respectively).

$$MU \sim \frac{V_m \times MP^x}{K_M + MP^x} \qquad (4),$$

with MU, urine value (which may represent the amount of vitamin C in urine (mg), urinary vitamin C excretion rate (mg/h) or urine vitamin C concentration in $\mu$M depending on the data set); $V_m$, maximum urine value (urine amount of vitamin C in mg, urinary vitamin C excretion rate in mg/h, or urine vitamin C concentration in $\mu$M); $K_M$, plasma concentration ($\mu$M) of vitamin C where the urine value/amount equals half of the maximum urine excretion rate/amount; MP, plasma concentration of vitamin C ($\mu$M) at time corresponding to the urine value; x, coefficient for MP.

Initial estimate of $V_m$ and $K_M$ were obtained by inspecting data plots. Goodness-of-fit was checked by visually inspecting the distribution of Standardized Residuals. If the goodness of fit was not acceptable, the initial parameters ($V_m$ and $K_M$) were changed and the resulting plot inspected again for distribution of residuals. This was repeated till the residuals were symmetrically distributed around zero without any discernible pattern. After computer iteration, final parameter values were obtained. In order to determine renal threshold, a cut off value at 5% (to account for experimental variation/errors) of the median urine value of urine vitamin C was used. The intersection of the fitted curve with the cutoff value was taken as the renal threshold for vitamin C.

*Comparison of methods.*

PBPK includes pharmacokinetic parameters generated for each individual using all the data set but may have errors due to model misspecification, although the model had been somewhat validated using independent datasets from other studies.

Population curve fitting incorporates data from all subjects to fit an empirical function to determine the population renal threshold for men or women. As a population measure, it conceals individual variations in the threshold but also minimizes the effect of individual experimental or measurements errors (called random errors) on the determination of renal threshold; a nonlinear mixed effects model analogue of the PBPK approach, one that may incorporate a statistical framework to account for uncertainty, subject-to-subject variability, and assess model misspecification (overcoming PBPK limitations) while also allowing for individual-level differences from a population-level parameter to accommodate shortfalls in either approach; however such alternate methods, whether based on measurement error or functional analysis typically require larger sample sizes than those conducted in studies to date, thus warranting evaluation of the need for such resource-intensive expanded depletion-repletion studies by first assessing associations between the working renal threshold and well-characterized clinical phenotypes, such as those presented herein.

The cross validation of the two methods added a good amount of certainty to the conclusions derived from the study.

**Minimal elimination threshold (MET)**

Minimal elimination threshold is defined as the plasma vitamin C concentration below which urinary vitamin C excretion would be inappropriate. At plasma vitamin C concentrations below the MET, renal tubular reabsorption of vitamin C will not be at maximum capacity and all filtered vitamin C should be completely reabsorbed resulting in no urinary excretion of vitamin C. To estimate the MET from the renal threshold, a similar approach used by the Institute of Medicine dietary reference intake guidelines in estimating recommended dietary allowance (RDA) of vitamins from the estimated average requirement (EAR) was applied. Using this approach, the MET was defined as 2 standard deviations below the mean renal threshold in men and women based on PBPK model.

**Vitamin C Renal Leak**

*Criteria*

When plasma vitamin C concentrations are below this MET, urinary vitamin C excretion would be inappropriate, a term referred to here as "renal leak." Renal leak criteria avoid the confounding issue of variable vitamin C dietary intake, as subjects who may have low plasma vitamin C levels due to dietary deficiency will only be considered "leakers" if they additionally have inappropriate urinary excretion of vitamin C for such low plasma vitamin C concentrations.

*Pre-study vitamin C dietary restriction in healthy controls*

Criteria for vitamin C renal leak (inappropriate urinary vitamin C excretion) consist of plasma vitamin C concentration below the MET and presence of urinary vitamin C excretion (urine vitamin C > 0.01mg). Thus, having similar plasma vitamin C concentrations in compared groups provide a more accurate comparison of vitamin C renal leak prevalence in both cohorts. Significantly higher plasma vitamin C concentrations in the healthy controls (above MET) would underestimate renal leak prevalence in healthy controls and result in a biased comparison with the Fabry's cohort.

*Matched urine and plasma vitamin C measurements*

Following an overnight fast, on the morning of study, subjects were instructed to empty their bladder. After a 1-hour interval, a spot urine sample and a sample of blood were obtained for measurement of fasting plasma and matched urine vitamin C concentrations (termed 1h urine), as well as chemistry and related studies. Following the initial spot urine collection, subjects were hospitalized for another 24 hours. They consumed a hospital diet free of supplements or foods items with high vitamin C content (such as orange juice). At the end of 24 hours (on the morning of day 2), collection all voided urine for 24 hours (termed 24h urine) was obtained and subjects were discharged home. Subjects could watch TV, read, take naps, or rest in any other way they choose. However, they were instructed not to do strenuous physical activity or exercise.

The use of fasting plasma vitamin C (no food after midnight the night before), was to ensure that measured plasma and urine vitamin C concentrations accurately reflected subjects' true baseline vitamin C status and not confounded by transient post-prandial changes that may result in inaccurate determination of renal leak status. Because the transient post-prandial rise in plasma vitamin C may not have fully equilibrated with urine excretion, determination of renal leak status using non-fasting vitamin C plasma concentrations would be inaccurate. Most subjects will return to their "baseline" plasma vitamin C concentrations by 8h, thus fasting plasma vitamin C will reflect the true baseline.

*Exploring Factors Predictive of Renal Leak Outcomes:*

As exhibited in Figure 4 and Table 3, a range of measured variables on both healthy male controls and men with Fabry disease was explored, and, in order to both exploit outcome variability and ensure adequately-powered detections of association, the two groups were pooled to leverage the full range their distinct distributions of renal leak outcomes (proportion prevalence, fractional excretion of vitamin C (FeVC), and - as a component of fractional excretion less typically measured clinically than creatinine - vitamin C concentration). In the case of continuous variables, bivariate scatterplots supplemented with data-driven smooths (and residuals from such splines) were employed to assess whether either or both of the possible predictor or outcome variables (as indicated) required transformation in order to meet regression assumptions (e.g., linearity on appropriate scale), to leverage regression estimates as a screening tool for associations (i.e., not be misled from findings that may be an artifact of model misspecification). In one instance, the key secondary endpoint's outcome of fractional excretion of vitamin C (FeVC): it was zero-inflated and highly skewed and was not amenable to a broadly applicable transformation (as explained in methods section of main manuscript, it lacked a Box-Cox power transformation parameter estimate consistent -- within sampling variability -- across 24-hour timepoints). Thus, in the case of comparing healthy male controls to men with Fabry disease, in order to corroborate the secondary endpoint of difference in medians using both conventional definitions and as estimated by quantile regression, a Wilcozon-Mann-Whiney test that the shift in location between the two distributions was different from zero also was employed, inspecting the corresponding Hodges-Lehmann point and interval estimates; findings were corroborated as shown in Figure 3F.

Once fitting models after these considerations, the screening for associations may have been prone to an inflated false positive (Type I family-wise error) rate and so an adjustment for multiplicity was applied within clinically consistent groupings: parallel adjustment via the Hommel procedure within each of demographics, renal function, and lab predictors, and parallel gatekeeping procedures using Hommel within each stage for the series of overall through to relative-to-MET subgroups, to allow for positive correlations among p-values of respective tests, as implemented in the multxpert package in R.

**Vitamin C in mouse models**

Animal experiments were approved by the Animal Care and Use Committee NIDDK, NIH, and were conducted in accordance with NIH guidelines. Mice were 10-14-week-old males and females with free access to food and water and were maintained on a regular chow diet (NIH-07) without detectable ascorbate (detection limit 10 nM). Mice type were gulo$^{-/-}$ and SVCT1$^{-/-}$. Gulo$^{-/-}$ mice do not have functional gulonolactone oxidase and are unable to synthesize vitamin C. To maintain the mice, vitamin C was provided via drinking water, which was changed daily. SVCT1$^{-/-}$ are homozygous knockout mice for sodium-dependent vitamin C transporter 1.

During the experiment, vitamin C at different doses was provided to SVCT1$^{-/-}$ and Gulo$^{-/-}$ mice via drinking water, which was changed daily. Plasma and RBCs were from mouse whole blood as described. Urine was collected at the same time as the blood by gentle abdominal pressure.

Blood and urine samples were collected on ice and processed promptly. All samples were stored at -80C and assayed together by HPLC as previously described.

**RESULTS**

***Determination of vitamin C renal threshold in healthy women and men: data from depletion-repletion studies***

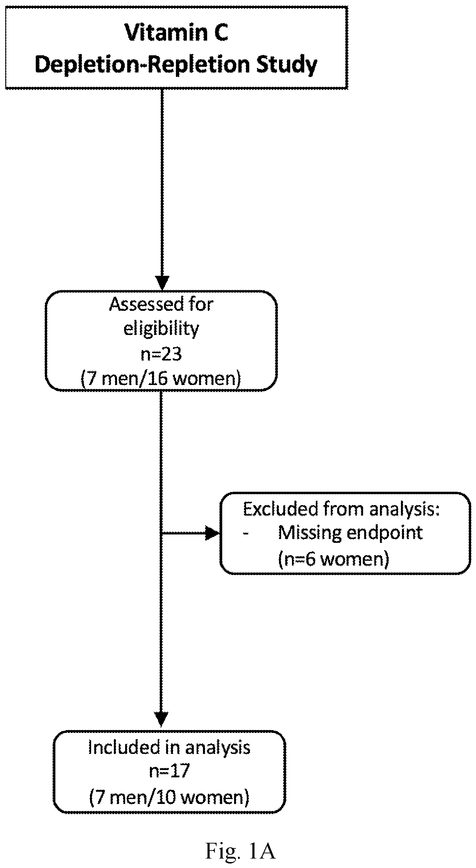

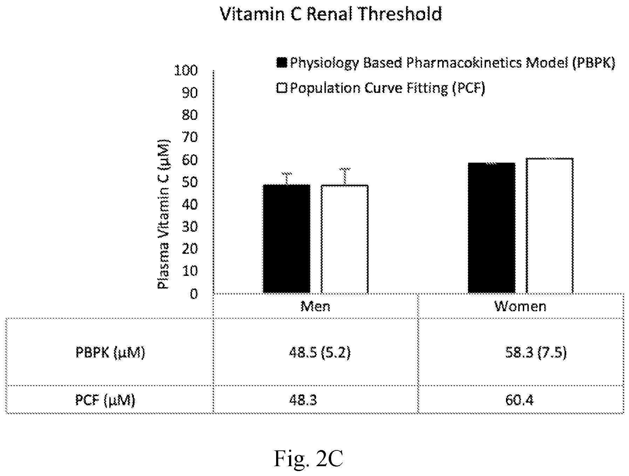

The first objective was to determine a normal vitamin C renal threshold: that plasma concentration above which vitamin C would be expected to be excreted in urine. Data from 17 healthy volunteers (7 men and 10 women) were included in the final analyses (Fig. 1A), and baseline characteristics of subjects are shown in Table. 1. Measurements of urinary vitamin C excretion as a function of matched plasma vitamin C concentrations are displayed for 7 men (Fig. 2A) and 10 women (Fig. 2B). Findings for men and women visually indicated a threshold plasma vitamin C concentration above which vitamin C was detected in urine. The renal threshold for men and women was calculated using two analytical models: a physiology-based pharmacokinetic model (PBPK), and a population curve-fitting (PCF) model (see methods and supplementary methods). Using the PBPK approach, a renal threshold of 48.5(5.2)$\mu$M for healthy men and 58.3(7.5) $\mu$M for healthy women was calculated (Fig. 2C, Table 2). The PCF approach revealed similar renal threshold values of 48.3$\mu$M in healthy men and 60.4$\mu$M in healthy women (Fig. 2C, Table 2). The renal threshold estimates in men and women were significantly different (P = 0.006, Figure 2C).

Next, the plasma vitamin C concentration below which urinary vitamin C excretion would be inappropriate, defined as 2SDs below the renal threshold for each sex, was determined. This concentration is termed the minimal elimination threshold (MET). Using the PBPK model, the MET was 38.1$\mu$M for healthy men and 43.2$\mu$M for healthy women (Fig. 2D, see methods). Taken together, when plasma vitamin C concentrations are less than the MET values of 38.1$\mu$M in men or 43.2$\mu$M in women, urinary vitamin C excretion would be inappropriate, and is termed a vitamin C renal leak (Fig. 2D).

***Vitamin C renal leak in Fabry's disease vs healthy controls***

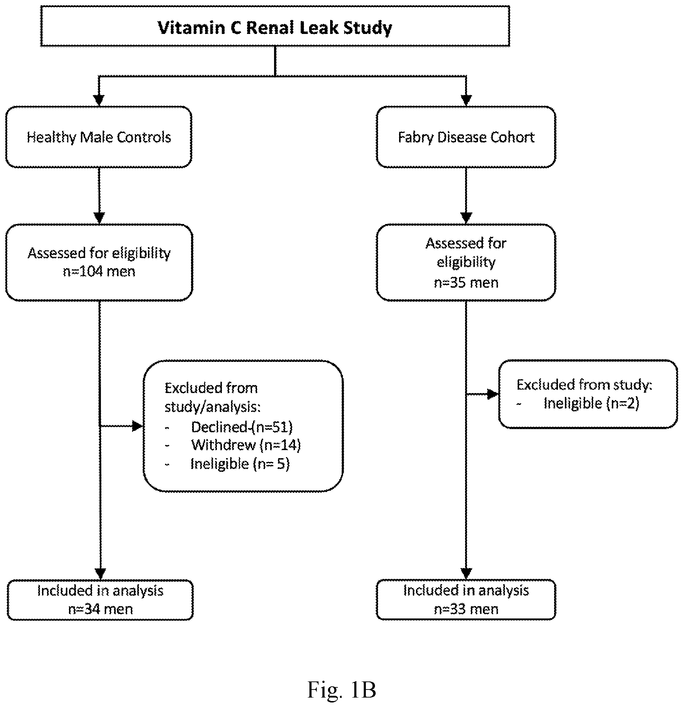

Using defined criteria for renal leak, the next objective was to investigate its prevalence and associated clinical characteristics in subjects with Fabry disease compared to healthy controls. Analyses included 33 men with Fabry's disease and 34 healthy men as controls (Fig.1B, baseline characteristics in Table. 1). Because Fabry disease is associated with low plasma vitamin C concentrations healthy controls were instructed to adhere to a low vitamin C diet 3 weeks prior to study sampling (see methods). As intended, the mean plasma vitamin C concentrations were similar in

both cohorts (38.9$\mu$M vs 36.5$\mu$M, p=0.6) (Figs. 5A-5C).

### *Vitamin C Renal Leak Prevalence*

Prevalence of renal leak was determined by measuring vitamin C in matched fasting plasma and timed 1-hour urine samples obtained from healthy men and men with Fabry disease (Figs. 3A and 3B). The data show that nearly all healthy men had no detectable vitamin C in urine when plasma concentrations were less that the MET (Fig. 3A). In contrast, nearly all men with Fabry disease who had plasma vitamin C concentrations less than the MET had detectable vitamin C in urine: a renal leak (Fig. 3B). The prevalence of vitamin C renal leak in both groups was calculated and compared. The vitamin C renal leak prevalence was 51.5% in the Fabry cohort compared with 5.9% in the healthy controls (Fig.3C, Figs. 5A-5C). Odds of having a vitamin C renal leak were approximately 16-fold higher for individuals with Fabry disease compared with healthy controls (p<0.001)(Fig. 3C). Data from 24-hour urine sample collections were also analyzed and compared to findings for 1-hour urine samples, with similar results (Fig. 5C).

### *Fractional excretion of vitamin C (FeVC)*

Fractional excretion of vitamin C (FeVC) was used to characterize and quantify the magnitude of renal leak. FeVC describes the proportion of urinary vitamin C excretion relative to plasma vitamin C concentrations corrected for creatinine for each subject (see methods). It was hypothesized that higher prevalence of vitamin C renal leak in Fabry disease vs healthy controls, as seen in Fig. 3C, would result in higher FeVC. The calculated FeVC for all subjects in the healthy group and Fabry disease cohort are shown in Figs. 3D and 3E, respectively. As expected in the healthy controls, FeVC values only exceeded zero at plasma vitamin C concentrations greater than the MET, a pattern not observed with the Fabry cohort. The median FeVC for the Fabry cohort was significantly higher than healthy controls (0.018vs 0, 95% confidence interval for difference in medians: 0.0093-0.0304; corroborated by a significant shift in distribution location per Wilcoxon-Mann-Whitney test, p=0.006)(Fig. 3F). This difference in median FeVC between healthy and Fabry men was stronger in subgroup with plasma vitamin C less than the MET (leakers) compared to those greater than the MET (Fig. 3F). These data are consistent with the hypothesis that subjects with a renal leak are the major drivers of quantitative urinary vitamin C loss in Fabry disease.

### *Clinical variables associated with vitamin C renal leak*

To better understand clinical implications of the dysregulated vitamin C physiology, multivariable exploratory analyses were performed to determine whether any clinical variables were predictive or associated with FeVC, a quantitative measure of renal leak (see statistical methods). Based on the premise that vitamin C renal leak is indicative of dysregulated vitamin C tubular reabsorption, it was hypothesized that FeVC would be associated with clinical variables related to renal function. This hypothesis was tested on all subjects regardless of disease status (Fig. 4). For renal function variables in both groups of subjects, increased FeVC was associated with decreased glomerular filtration rate (eGFR), increased protein creatinine ratio (PCR), and protein excretion (mg protein per 24h). Use of diuretics, angiotensin converting enzyme (ACE) inhibitors or angiotensin II receptor blockers (ARBs) were not associated with FeVC. Of the other clinical variables assessed for all subjects, increased FeVC was associated with increased aspartate aminotransferase (AST) and decreased hemoglobin levels.

### *Vitamin C renal threshold in animal models*

Due to the presence of vitamin C renal re-absorptive transporters in other species, whether a renal threshold for vitamin C would be found in animals was investigated. Because most animals synthesize vitamin C, a mouse model was used in which the last step in vitamin C biosynthesis was knocked out (gulo$^{-/-}$ mice). These mice do not have distinctive pathologies and have normal survival when vitamin C is provided, usually in drinking water. First it was investigated and determined that gulo$^{-/-}$ mice have tight control of vitamin concentrations as a function of ingested dose of vitamin C. Plasma concentrations at saturation are similar to those found in humans (Fig. 6A). Next, it was found that a renal threshold for vitamin C occurs in gulo$^{-/-}$ mice of both sexes. No vitamin C was found in urine at plasma vitamin C concentrations less than 55 $\mu$M (Fig. 6B). Renal reabsorption of vitamin C is dependent in part on sodium vitamin C transporter 1. It was hypothesized that if SVCT1 was dysfunctional, relationships would be disrupted between vitamin C doses ingested, plasma vitamin C concentrations, and vitamin C urine excretion (Figs. 6C and 6D). Using SVCT1 knockout mice, it was found that renal threshold was absent, and plasma concentrations did not achieve saturation concentrations; instead there was continuous renal leak of vitamin C (see Fig. 6A). Together, these data suggest that a renal threshold is part of normal vitamin C physiology in mammals and is not specific to humans.

## DISCUSSION

The data in this Example, the first of their kind, define normal physiologic parameters associated with renal regulation of vitamin C in healthy men and women. Using depletion-repletion studies, normal vitamin C renal threshold: the plasma vitamin C concentration above which urinary excretion of vitamin C occurs in men and women, was estimated. The minimal elimination threshold (MET): the plasma vitamin C concentrations below which urinary vitamin C excretion would be inappropriate and considered a renal leak, also was determined. These physiologic parameters were integrated into a simplified criteria for determining when urinary vitamin C excretion is appropriate or inappropriate at a given plasma vitamin C concentration-criteria for renal leak. Characterization of vitamin C physiology in healthy people provided the foundation for exploring vitamin C pathophysiology in disease states such as Fabry disease.

In the cross-sectional cohort study, men with Fabry disease had significantly increased prevalence of vitamin C renal leak compared with healthy men. This increased renal leak prevalence was associated with higher fractional excretion of vitamin C (FeVC). The results also showed that in a mouse model, knockout of a vitamin C renal reabsorption transporter recapitulated the clinical renal leak observations.

Taken together, these findings suggest that vitamin C renal leak in Fabry disease is indicative of aberrant vitamin C tubular reabsorption. However, mechanisms of tubular reabsorption of vitamin C have not fully characterized. Vitamin C transporter SLC23A1 (SVCT1) transports vitamin C from the tubule lumen into the tubular cell, analogous to tubular glucose reabsorption by SLC5A2 (SGLT2). Disruptions of vitamin C transport either into or out of tubular cells may be a potential mechanism for renal leak, however further translational research is required to test this hypothesis. A renal leak could be substantial enough to lower plasma and tissue vitamin C concentrations, one potential explanation for the low vitamin C concentrations observed in Fabry disease. Reduced vitamin C concentrations resulting from renal leak might reduce antioxidant capacity which may have implications in disease pathophysiology and pathogenesis.

Results showed that reduced glomerular filtration rate and increased proteinuria were significantly predictive of increased FeVC--a quantitative measure of vitamin C renal leak. The findings suggest that vitamin C renal leak is an indicator, predictor or biomarker of disease status or progression. This is particularly pertinent in Fabry disease, because new biomarkers are needed to guide when to initiate enzyme replacement therapy that is both intensive and expensive. Given the multi-organ nature of Fabry disease, it is plausible that individuals with a renal leak will also have related comorbidities consistent with progressing Fabry disease. Beyond Fabry disease, vitamin C renal leak as a diagnostic tool or biomarker can be used in the context of a broad range of diseases that have renal involvement, such as diabetes and metabolic diseases; infectious, inflammatory and autoimmune syndromes.

Previous characterizations of a renal excretion of vitamin C in humans are surprisingly limited and conflicting, due to insensitive and non-specific vitamin C measurement techniques coupled to lack of subjects with a broad range of vitamin C plasma concentrations. Soon after discovery of vitamin C, clinical experiments to determine its renal excretion indicated that vitamin C was lost in urine at all plasma concentrations. In those and subsequent investigations, the vitamin C assay used was non-specific, with interference from other reducing compounds especially at low concentrations. These interferences gave a false positive signal for presence of vitamin C in urine, so that an accurate renal threshold could not be calculated. Similarly, these interferences produced artificially elevated and perplexing measurements of plasma vitamin C in balance studies where subjects had clear clinical scurvy. Prior to the experiments presented here, no studies had subjects with vitamin C concentrations over a broad range and with sufficiently low values, nor were measurements performed using a highly sensitive and specific assay without confounding interferences.

Table 1

| | Vitamin C Depletion/Repletion | Vitamin C renal leak study | |
| --- | --- | --- | --- |
| | | Healthy control | Fabry disease |
| | n=17 | n=34 | n=33 |
| **Demographics** | | | |
| Ethnicity n (%) | | | |
| White/Caucasian | 15 (88%) | 20 (59%) | 33 (100%) |
| Black/African American | 0 (0%) | 8 (23%) | 0 (0%) |
| Other[1] | 2 (12%) | 6 (15%) | 0 (0%) |
| Sex n (%) | | | |
| Male | 7 (41%) | 34 (100%) | 33 (100%) |
| Female | 10 (59%) | 0 (0%) | 0 (0%) |
| Age (years), Median (IQR) | 22 (21-23) | 30.5 (24-39) | 39 (30-42) |
| BMI (Kg/m²) Mean (SD) | 26 (11.5) | 26 (3.8) | 25.6 (4.9) |
| **Clinical and Lab Variables** | | | |
| Renal Function | | | |
| PCR[2,3] (mg/g) Mean (SD) | | 76 (49.5) | 755 (1061) |
| Protein Excretion[3] (mg/24h) Mean (SD) | | 135 (101) | 1110.3 (1685.2) |
| eGFR[4] (mL/min/1.73m²) Mean (SD) | 94 (21.2) | 100 (17.6) | 78.9 (32.7) |
| ACE/ARB/Diuretic use (%) | 0% | 0% | 30% |
| Lab Predictors Mean (SD) | | | |
| ALT (U/L) | 18 (6.5) | 27 (7.5) | 20.2 (7.7) |
| AST (U/L) | 20 (4.3) | 20 (6.2) | 21.4 (5.9) |
| Alkaline Phosphatase (U/L) | 71 (12.7) | 66 (17.3) | 77 (18.9) |
| Fasting Glucose (mg/dl) | 88 (4.6) | 87 (6.3) | 92.3 (5.9) |
| Hemoglobin (g/dl) | 13.7 (1.2) | 15 (1) | 13.6 (1.4) |

[1]The subgroup "Other" is composed of Asians, Hispanic and Latino and multiethnic subjects.
[2]PCR reference range (<200mg/g).
[3]Urine protein raw values for these patients were non available (under the limit of detection).
[4]eGFR for all group was calculated using the Modification of Diet in Renal Disease v4 (MDRD4) equation: [186 x (Creatinine/88.4)-1.154 x (Age)-0.203 x (0.742 if female) x (1.210 if black)].
Abbreviations: BMI, body mass index; PCR, protein creatinine ratio; ACE, angiotensin-converting enzyme inhibitor; ARB, Angiotensin Receptor Blocker; AST, aspartate aminotransferase; ALT, alanine aminotransferase

Table 2

Individual and group mean estimates of vitamin C renal threshold from depletion-repletion studies

A

| Subject ID (Men) | Pharmacokinetic Modeling (PBPK) | Population Curve Fitting (PCF) |
|---|---|---|
| M1 | 50 μM | |
| M2 | 45 μM | |
| M3 | 52 μM | |
| M4 | 51 μM | |
| M5 | 40 μM | 48.28 μM |
| M6 | 56 μM | |
| M7 | 45 μM | |
| **Mean (SD)** | **48.5 (5.2) μM** | |

B

| Subject ID (Women) | Pharmacokinetic Modeling (PBPK) | Population Curve Fitting (PCF) |
|---|---|---|
| W1 | 54 μM | |
| W2 | 67 μM | |
| W3 | 57 μM | |
| W4 | 64 μM | |
| W5 | 63 μM | |
| W6 | 54 μM | 60.39 μM |
| W7 | 45 μM | |
| W8 | 70 μM | |
| W9 | 54 μM | |
| W10 | 55 μM | |
| **Mean (SD)** | **58.3 (7.5) μM** | |

Table 3

Association between Clinical Variables and Fractional Excretion of Vitamin C in all subjects Numerical value for Figure 4

| | Estimated Shifts | Confidence Interval Upper | Lower | p-value: Unadjusted [*Adjusted within Variable group*] |
|---|---|---|---|---|
| **Demographics** | | | | |
| Age | 0.000163 | -0.00011 | 0.000439 | 0.244 [0.3216] |
| BMI | 0.000317 | -0.00032 | 0.000952 | 0.3216 [0.3216] |
| **Renal Function** | | | | |
| eGFR | -0.00021 | -0.00029 | -0.00013 | 3.62E-6 [3.62E-6] |
| PCR | 0.0569 | 0.0272 | 0.0866 | 0.000299 [0.0009] |
| Protein Excretion | 0.0343 | 0.0147 | 0.0539 | 0.000889 [0.0018] |
| ACE/ARB/Diuretics | 0.0000822 | -3.7E-06 | 0.0001681 | 0.059987 [0.0600] |
| **Lab Predictors** | | | | |
| Glucose | 0.000346 | -5.34E-05 | 0.000746 | 0.0883 [0.2648] |
| AST | 0.000524 | 0.0001 | 0.000948 | 0.0162 [0.0810] |
| ALT | -0.00016 | -0.00048 | 0.000165 | 0.3310 [0.3896] |
| Alkaline Phosphatase | 6.12E-05 | -8.00E-05 | 0.000202 | 0.3896 [0.3896] |
| Hemoglobin | -0.00003 | -0.000048 | -0.000012 | 0.0018 [0.0106] |
| Hematocrit | -.000058 | -0.000128 | 0.000011 | 0.0992 [0.2975] |

EXAMPLE 2

This Example demonstrates the presence of vitamin C renal leak and low plasma vitamin C concentrations in patients with diabetes. Abbreviations used herein include NIH CRC: National Institutes of Health Clinical Research Center; MET: Minimal Elimination Threshold; FeVC: Fractional Excretion of Vitamin C; and RDA: Recommended Dietary Allowance.

Briefly, this cross-sectional cohort study of 162 individuals; 82 with diabetes and 80 nondiabetic controls, investigated the prevalence and clinical characteristics of vitamin C renal leak, using fasting plasma and matched 1h urine samples. Based on vitamin C renal threshold, vitamin C renal leak was defined as presence of vitamin C in urine with plasma concentrations less than the minimal elimination threshold (MET) of 43.2 $\mu$M in women or 38.1 $\mu$M in men (see Example 1). The median fractional excretion of vitamin C (FeVC) and the mean plasma vitamin C concentrations between groups also were compared. Vitamin C in plasma and urine was measured using coulometric electrochemical detection with high performance liquid chromatography.

When compared with nondiabetic controls, subjects with diabetes had significantly increased prevalence of vitamin C renal leak (8.8% vs 32.9%: OR5, p<0.001) and decreased mean plasma vitamin C concentrations (53.1 $\mu$M vs 40.9$\mu$M, p<0.001). At plasma vitamin C less than MET, diabetic subjects had increased FeVC compared with nondiabetic controls. Higher fasting plasma glucose, glycosylated hemoglobin A1c, body mass index, micro/macrovascular complications, and protein creatinine ratio were predictive of vitamin C renal leak. These findings reveal that increased prevalence of vitamin C renal leak in diabetes is associated with increased fractional excretion of vitamin C, and reduced plasma vitamin C concentrations. Glycemic control, microvascular complications, obesity and proteinuria are predictive of renal leak.

INTRODUCTION

Diabetes is a chronic disease characterized by microvascular and macrovascular angiopathies. Compared to healthy populations, vitamin C concentrations are lower in people with diabetes. Unfortunately, many studies of vitamin C in diabetes are limited by ambiguous dietary control, poor vitamin C assay accuracy and precision, and flawed methods for measurement of vitamin C metabolites. Thus, based on current understanding, vitamin C concentrations in diabetes could be explained by poor dietary intake, decreased bioavailability, and increased utilization. A major mechanism that is inadequately characterized is the possibility of increased urinary loss of vitamin C in diabetes.

Example 1 explored the renal leak in a cross-sectional cohort study of men with Fabry disease, an X-linked lysosomal storage disorder associated with low plasma vitamin C concentrations. As noted in Example 1 above, increased prevalence of vitamin C renal leak in Fabry disease compared to healthy controls was observed. Vitamin C renal leak was also associated with abnormally increased fractional excretion of vitamin C (FeVC), a quantitative measure of renal leak. Reduced renal function and proteinuria were predictive of increased fractional vitamin C excretion.

It is unknown if a similar pattern of vitamin C dysregulation occurs in diabetes. The prevalence of a vitamin C renal leak in diabetes and the clinical implications for individuals with diabetes are also unknown. Fabry disease and diabetes are both chronic conditions with renal complications. The pathophysiology in both diseases involve renal tubules where vitamin C is reabsorbed, and both are associated with low plasma vitamin C concentrations. However, unlike the rare condition of Fabry disease, diabetes and its complications are common. For a highly prevalent disease such as diabetes, knowledge of a disease-related correctable vitamin insufficiency would be relevant for public health. Furthermore, an understanding of potential dysregulation in vitamin C renal physiology associated with diabetes is an essential pre-requisite for developing corrective and/or preventative nutritional strategies.

In this Example, the highly sensitive and specific vitamin C assay discussed in Example 1 is used to evaluate the prevalence and clinical characteristics of vitamin C renal leak in diabetic subjects and nondiabetic controls. Clinical and laboratory parameters associated with a vitamin C renal leak also are investigated.

METHODS

*Study design*: This was a non-randomized cross-sectional cohort outpatient study aimed at investigating the prevalence and clinical characteristics of vitamin C renal leak in a cohort of individuals with diabetes and non-diabetic controls. The protocol was approved by the Institutional Review Boards of NIDDK, NIH.

*Participants and Recruitment*: The study included a cohort of diabetic and non-diabetic individuals aged 18-65, without known acute or chronic medical conditions other than complications of diabetes mellitus or metabolic syndrome. Exclusion criteria included presence of an acute or chronic illness, alcohol abuse or tobacco use and pregnancy. The exclusion criteria were chosen to avoid conditions that may confound study findings, given reported association of these conditions with vitamin C dysregulation. Recruitment was conducted using recruitment flyers and advertisements displayed in the community, social media, and online registries.

*Screening Evaluation*: Interested subjects were pre-screened by telephone and completed a structured questionnaire on their medical history. Eligible subjects were then scheduled for an outpatient screening visit at the NIH CRC for a more detailed clinical history and physical exam by research investigators. Subjects who met screening criteria were scheduled for the protocol sampling studies on a rolling basis to accommodate use of the CRC's metabolic unit.

*Study Interventions:* Approximately 3-4 weeks prior to study sampling, subjects were instructed to avoid vitamin C supplements or foods items with very high vitamin C content (such as orange juice). Subjects were also instructed to avoid food or drinks (other than water and medications) after midnight, prior to study sampling day. On morning of study sampling, subjects were admitted to the NIH CRC's metabolic unit as outpatients. They were instructed to empty their bladder. After a 1hour interval, matched urine and fasting blood samples were obtained for measurement of vitamin C concentrations, chemistry, and related studies. Subjects were subsequently provided with a meal and discharged home.

*Study Groups and Comparators for Analyses:* Three groups of participants who met the inclusion and exclusion criteria were used for data analyses: diabetic cohort, nondiabetic control group and healthy subgroup. The *diabetic cohort* was comprised of men and women with type 1 or type 2 diabetes. The *nondiabetic control group* was made up of individuals without diabetes and were not taking any chronic medications. To investigate unknown factors associated with renal leak, related and unrelated to diabetes, the study exclusion criteria was non-restrictive to recruit a broad sampling of the population. Thus, the nondiabetic control group included asymptomatic subjects with abnormal laboratory values which not determined to be clinically significant during the screening evaluation. However, inclusion of individuals with abnormal laboratory values in the non-diabetic control group could confound study findings. To address this concern, a post-hoc *healthy subgroup* of the nondiabetic control group without abnormal laboratory findings was identified. Primary comparative analysis was between the diabetic group vs nondiabetic control group. The diabetic group was secondarily compared with the healthy subgroup to assess for discordant findings.

### Study Outcomes and Endpoints

*Primary Outcomes:* The two co-primary outcomes were: (1) difference in prevalence of vitamin C renal leak in diabetic subjects compared with non-diabetic controls, and (2) difference in median fractional excretion of vitamin C between groups.

*Vitamin C renal leak* was defined as the inappropriate urinary excretion of vitamin C at plasma concentrations below the minimal elimination threshold (MET) of 43.2 $\mu$M in women and 38.1 $\mu$M in men (Example 1). The MET and vitamin C renal leak criteria were derived using data from vitamin C depletion-repletion studies and statistical modeling (Example 1). Using the renal leak criteria, subjects were classified as "leakers" or "non-leakers" based on fasting plasma and matched urine vitamin C concentrations. This binary criterion is a qualitative measure indicating the presence or absence of a renal leak in a subject. The prevalence of renal leak was calculated as the proportion of leakers in each population cohort.

*Fractional Excretion of Vitamin C (FeVC)* is a ratio describing the proportion of urinary vitamin C excretion as a function of plasma vitamin C concentration, adjusted by creatinine. FeVC is a quantitative measure that describes the degree and magnitude of a renal leak. The formula for calculating FeVC is shown below:

$$A = \frac{\dfrac{[urine\ ascorbate\ concentration\ (\mu M)] * [urine\ volume\ (l)]}{[plasma\ ascorbate\ concentration\ (\mu M)]}}{\dfrac{[urine\ creatinine\ concentration\ (\mu M)] * [urine\ volume\ (l)]}{[plasma\ creatinine\ concentration\ (\mu M)]}}$$

*Secondary Outcomes:* The secondary outcomes included comparisons of the mean difference in plasma vitamin C concentrations between groups. Secondary outcomes also assessed the demographic, clinical and laboratory covariates associated with vitamin C renal leak.

*Sample collection and vitamin C assay:* Urine and plasma vitamin C were analyzed by HPLC with coulometric electrochemical detection. Other blood and urine chemistry measurements were performed, using standard laboratory methods, by the department of laboratory medicine Clinical Laboratory Improvement Amendments (CLIA) identification number: 21D0665373, Clinical Research Center, National Institutes of Health.

*Sample Size:* At time of initial study design, there were no real data to base a sample size calculation on. Sample size calculations were based on scant anecdotal evidence indicating substantial differences in 24-hour urinary vitamin C excretion between healthy controls and diabetic subject. It was therefore assumed that magnitude of differences will be between Cohen's "medium" and "large" differences, corresponding to an effect size between 0.5 and 0.8; value of 0.65 was chosen. Using a roughly a 2:1 recruitment goal and standard 2-sample t-test, at a 2-sided significance level of 0.05, numbers of 30 non-diabetic controls and 60 diabetic subjects were projected to yield statistical power of approximately 80 or 81% (Cohen table 2.3.5). It was determined that the estimated sample size necessary to determine whether diabetic subjects lose more vitamin C in the urine than normal controls will be approximately 150.

*Statistical Analyses Plan*: Group-specific estimates and comparative measures (e.g., odds ratios) were accompanied by their corresponding (large-sample normal approximation-based) 95% confidence intervals and p-values unless otherwise indicated. In the case of estimated proportions such as renal leak prevalence, an exact binomial confidence interval designed to match a 'central' Fisher's Exact Test was employed. When screening multiple variables for association with renal leak measures, multiplicity adjustments were not performed to control overall familywise (Type I) error rates, as these were considered exploratory outcomes.

## RESULTS

### Characteristics of Study Cohorts

177 individuals were assessed for eligibility, 15 subjects were excluded due to ineligibility. 162 subjects completed the protocol study, 80 subjects with diabetes and 82 non-diabetic controls. Among the nondiabetic control group, 26 subjects with no abnormal laboratory values were used as a healthy subgroup (see methods and Fig. 7). All subjects who participated in protocol studies were included in final analyses. The demographic, clinical and laboratory characteristics of individuals in the diabetic group, non-diabetic controls and healthy subgroup are shown in Table 4. Both primary groups had a similarly diverse ethnic makeup. Compared with the diabetic group, the nondiabetic control group included more proportion of women (63% vs 51%), younger population (34 vs 49years) (Table 4).

### Plasma Vitamin C Concentrations and Renal Leak Prevalence

Vitamin C renal leak was defined as the presence of inappropriate urinary vitamin C excretion at plasma vitamin C concentrations below the minimum elimination threshold (MET) values of 38.1$\mu$M in men and 43.2$\mu$M in women (See Example 1). Vitamin C renal leak status was determined using vitamin C measurements in matched fasting plasma and 1hour timed urine samples. More subjects with diabetes had renal leak compared with nondiabetic control group, and no subjects in the healthy subgroup had a renal leak (Figs. 8A-8C).

The prevalence of vitamin C renal leak in diabetic subjects and non-diabetic controls was calculated and compared. The prevalence of vitamin C renal leak in the diabetic cohort was significantly higher compared with nondiabetic controls (32.9% v 8.8%, OR5.1 p<0.01) and healthy subgroup (32.9% v 0%, OR5.5, p<0.001) (Fig.8D, Table 5, part A). It was hypothesized that increased renal leak prevalence would be associated with decreased mean plasma vitamin C concentrations. Data showed significantly lower mean plasma vitamin C concentrations in diabetic subjects compared to either non-diabetic controls (40.9$\mu$M v 53.1$\mu$M, p<0.001) or the healthy subgroup (40.9$\mu$M v 57.1$\mu$M, p<0.001) (Fig.8E, Table 5, part B).

### Fractional excretion of vitamin C (FeVC)

The fractional excretion of vitamin C (FeVC), a quantitative measure of renal leak that describes the proportion of urinary vitamin C excretion relative to plasma vitamin C concentrations corrected for creatinine for each subject (see methods) was calculated and compared. It was hypothesized that higher prevalence of vitamin C renal leak in the diabetes cohort would be associated with higher FeVC. FeVC values for all subjects are shown in Fig.9A-9C. In the healthy subgroup with 0% renal leak, FeVC values only exceeded zero at plasma vitamin C concentrations greater than the MET for men and women, a pattern not observed in either the nondiabetic controls or the diabetic cohort with renal leak of 8.8% and 32.9% (Fig. 8D, 9A-9C). When compared, it was found that the median FeVC in the diabetic group was not significantly different than the nondiabetic controls (Fig. 9D). However, when between-group comparisons are stratified by plasma concentrations less than the MET (leakers), the median FeVC in the diabetic group was significantly higher compared with nondiabetic controls (Fig. 9D). Comparison between the diabetic cohort and healthy subgroup revealed similar findings. Taken together, data suggest that diabetic subjects with a renal leak have increased FeVC compared to diabetic without a renal leak.

### Clinical variables associated with vitamin C renal leak

To investigate the clinical implications of the dysregulated vitamin C physiology in subjects with and without diabetes, multivariable exploratory analyses were performed to determine whether any clinical variables were predictive or associated with vitamin C renal leak. The hypothesis was tested on all subjects regardless of disease status (Fig.10, Table 7.). Findings revealed that BMI, glycemic parameters of glucose and hemoglobin A1c, and history of micro/-

macrovascular complications, were predictive of renal leak. Diabetes type (type 1 compared with type 2), duration, eGFR, use of diuretics, and angiotensin-converting enzyme inhibitors/angiotensin receptor blockers, ethnicity, sex, and age were not associated with renal leak. Additionally, protein creatinine ratio, but not glomerular filtration rate was associated with renal leak.

DISCUSSION

This cross-sectional cohort study provides the first characterization of vitamin C renal leak in diabetes, and its association with low plasma vitamin C concentration. These findings showed that diabetic subjects had significantly higher prevalence of vitamin C renal leak (33.9%) compared with nondiabetic controls (8.8%) and healthy subgroup (0%). Mean fasting plasma vitamin C concentration was also significantly lower in the diabetic cohort (40.9$\mu$M) compared with nondiabetic controls and healthy subgroup (53.1 $\mu$M and 57.1$\mu$M respectively). Findings also showed that at plasma vitamin C concentrations less than the minimal elimination threshold (MET), diabetic subjects had increased fractional excretion of vitamin C (FeVC) compared with nondiabetic controls and healthy subgroup. Clinical parameters predictive of a renal leak included fasting glucose, hemoglobin A1c, body mass index (BMI) and protein creatinine ratio (PCR). Age, gender, and ethnicity were not associated with renal leak.

These findings support the hypothesis that increased prevalence of renal leak is associated with low plasma vitamin C concentrations observed in individuals with diabetes. Vitamin C renal leak not only describes the presence of low plasma vitamin C concentrations less than the MET; renal leak additionally describes the inappropriate excretion of vitamin C for such low plasma vitamin C concentration, which is indicative of dysregulated vitamin C renal physiology.

Fractional excretion of vitamin C (FeVC) is a ratio that expresses the amount of vitamin C urinary excretion as function of plasma concentration adjusted by creatinine clearance. Unlike the binary renal leak criteria that qualitatively describes the presence of absence of a renal leak, FeVC is a quantitative measure that describes the magnitude of a renal leak. In healthy individuals, FeVC would be zero at plasma vitamin C concentrations less than the MET, as all the filtered vitamin C in the glomerulus would be reabsorbed by the renal tubules and not excreted. Thus, the significantly higher median FeVC in the diabetic group compared with nondiabetic controls at plasma concentrations less than, but not greater than the MET, suggests that the increased pool of diabetic leakers within that subgroup are primarily driving the abnormally elevated FeVC. Furthermore, these findings suggest renal leak may be indicative of aberrant renal function in diabetes, or diabetic nephropathy. Consistent with this possibility, renal leak was also associated with proteinuria, an indicator of diabetic nephropathy.

In diabetes, this relationships between vitamin C renal leak, low plasma vitamin C concentrations and diabetic nephropathy are also modulated by glycemic control, insulin resistance and metabolic syndrome. The present study showed that elevated fasting glucose, hemoglobin A1c and BMI were all predictive of a renal leak. Chronic hyperglycemia, insulin resistance and obesity are associated with increased oxidative stress. Increased oxidative stress contributes to microvascular complications such as diabetic nephropathy. For a disease characterized by increased oxidative stress, presence of a vitamin C renal leak might exacerbate an already diminished antioxidant capacity with implications for disease pathogenesis. This disease-induced increase in nutritional requirement represents a form of relative nutritional deficiency. Recent studies have demonstrated improved albuminuria, and glycemic parameters following oral vitamin C supplementation. However, larger studies that account for renal leak status might identify individuals or cohorts who could benefit from early and regular vitamin C supplementation.

Vitamin C undergoes glomerular filtration and is reabsorbed in renal tubules, most likely proximal tubules. Findings from vitamin C transporter-knockout mouse and in men with Fabry disease (Example 1) suggest disrupted renal tubular reabsorption as a mechanistic basis for a renal leak. In diabetes, there is both tubular dysfunction and glomerular dysfunction. Proteinuria is considered a marker of glomerular dysfunction. In contrast, measures of tubular dysfunction are less certain. While markers of tubular function of have been proposed, it is unclear whether they can be applied to diabetes, and they are not used widely. Vitamin C renal leak has potential to be a new marker of renal dysfunction in diabetes, and in other renal diseases.

Given the cross-sectional study design, the renal leak measurements described in this Example are single snapshots in time. These findings suggest that vitamin C renal leak might precede proteinuria in some populations and is an earlier marker of diabetic nephropathy compared to proteinuria. Accordingly, therapeutic interventions with ACEs, ARBs, SGLT2 inhibitors, and other agents based on vitamin C renal leak can have enhanced effects to delay nephropathy compared to use of these agents based on appearance/progression of proteinuria.

Table 4

| Baseline Characteristics of Non-diabetic and diabetic participants | | | |
|---|---|---|---|
| | Non-Diabetic Cohort | Healthy Subgroup | Diabetic Cohort |
| | N=80 | N=26 | N=82 |
| **Demographics** Race [n(%)] | | | |
| White/Caucasian Black/African American Other | 35 (43.8%) 36 (45%) 9 (11.2%) | 16 (61.5%) 8 (30.8%) 2 (7.7%) | 34 (41.5%) 36 (43.9%) 12 (14.6%) |
| Gender [n(% )] | | | |
| Male Female | 30 (37.5%) 50 (62.5%) | 9 (34.6%) 17 (65.4%) | 40 (48.8%) 42 (51.2%) |
| Age (years), Mean (SD) | 33.5 (12.6) | 25.8 (5.6) | 49.2 (12.1) |
| BMI (Kg/m $^2$) Mean (SD) | 27.5 (5.7) | 23.8 (2.6) | 32.5 (6.7) |
| **Clinical and Lab Variables** | | | |
| Renal Function [Mean (SD)] | | | |
| PCR (mg/mg) | 0.097 (0.091) | 0.070 (0.054) | 0.267 (0.557) |
| eGFR[1] (mL/min/1.73m$^2$) | 112.8 (21.1) | 116.2 (23.5) | 101.6 (26.2) |
| Glycemic Control [Mean (SD)] | | | |
| Fasting Plasma Glucose(mg/dl) | 88.3 (8.8) | 84.5 (6.9) | 147.0 (53.3) |
| Hemoglobin A1C (%) | 5.37 (0.4) | 5.1 (0.4) | 7.9 (1.4) |
| Lipid/Cholesterol [Mean (SD)] | | | |
| Total Cholesterol (mg/dl) | 169.3 (39.3) | 148.3 (16.5) | 172.7 (37.8) |
| LDL (mg/dl) | 92.3 (37.3) | 70.4 (17.5) | 94.16 (33.6) |
| HDL (mg/dl) | 58.6 (15.8) | 64.1 (15) | 54.84 (16.6) |
| Triglyceride (mg/dl) | 81.9 (53.7) | 68.6 (27.3) | 121.6 (83.9) |
| Liver Function | | | |
| AST (U/L) | 18 (8.6) | 20.1 (4.9) | 22.1 (13.6) |
| ALT (U/L) | 20 (4.6) | 16.2 (6.4) | 25.7 (15.6) |
| Alkaline Phosphatase (IU/L) | 63.3 | 52.7 (12.4) | 80.5 (22.1) |
| Hematological | | | |
| Hemoglobin (g/dl) | 14.3 (9.3) | 13.5 (1.0) | 12.9 (1.5) |
| Cardiovascular Risk | | | |
| 10-year Risk Score (%) | 2.88 (2.33) | NA[4] | 10.77 (7.30) |
| Lifetime Risk score (%) | 24.62 (15.22) | 16.1 (11.8) | 23.45 (17.82) |
| Diabetes Hi story | | | |

(continued)

**Clinical and Lab Variables**

| | | | |
|---|---|---|---|
| Diabetes Type N (%) | NA[5] | NA[5] | Type 1: 21 (25%)<br>Type 2: 59 (70.2%) |
| Duration of Diagnosis Mean (SD) | NA[5] | NA[5] | 11.5 (11.3%) |
| Micro or Macrovascular disease N (%) | NA[5] | NA[5] | 20 (23.8%) |
| Medication Antihypertensives | NA[5] | NA[5] | 45 (53.6%) |
| Diuretics[6] | NA[5] | NA[5] | 15 (17.9%) |
| ACE/ARB | NA[5] | NA[5] | 41 (48.8%) |
| Statin therapy | NA[5] | NA[5] | 31 (36.9%) |

[1]eGFR for all group was calculated using the Modification of Diet in Renal Disease v4 (MDRD4) equation: [186 x (Creatinine/88.4)-1.154 x (Age)-0.203 x (0.742 if female) x (1.210 if black)]. Cardiovascular Risk was calculated using the American Heart Association and American College of Cardiology (cvriskcalculator.com). [4] Variable for Diabetic Cohort only. [5] Diuretics includes loop diuretics, thiazides, aldosterone antagonists. Abbreviations: BMI, body mass index; PCR, protein creatinine ratio; LDL, Low Density Lipoprotein; HDL, High Density Lipoprotein; AST, aspartate aminotransferase; ALT, alanine aminotransferase. ACE, angiotensin-converting enzyme inhibitor; ARB, Angiotensin Receptor Blocker.

Table 5

Vitamin C renal leak prevalence in the diabetic cohort, nondiabetic controls and health subgroup. Numerical values for Figures 8A-E

**A- Vitamin C Renal Leak Prevalence in Diabetic cohort vs Nondiabetic control**

| Groups | Renal Leak Prevalence (%) | Odds Ratio (vs Diabetic Cohort) | CI, min | CI, max | p-Values |
|---|---|---|---|---|---|
| Diabetic Cohort | 32.9% | NA | | | |
| Non-Diabetic Control | 8.8% | 5.07 | 1.970207 | 14.83355 0 | .0002461 |
| Healthy Subgroup | 0.0% | 5.51 | 2.037511 | 17.49274 0 | .0002151 |

**B - Plasma Vitamin C in Diabetic cohort vs Nondiabetic control (Un-Adjusted for sex/Gender)**

| Groups | Plasma Vitamin C Concentration | Mean Difference C (vs Diabetic Cohort) | CI, min | CI, max | p-Values |
|---|---|---|---|---|---|
| Diabetic Cohort | 40.9 $\mu$M | NA | | | |
| Non-Diabetic Control | 53.1 $\mu$M | -12.20 | -18.393 | 9 6.0227 | 0.00014 |
| Healthy Subgroup | 57.1 $\mu$M | -12.67 | -18.921 | 2 6.4266 | 0.0001 |

**C - Plasma Vitamin C in Diabetic cohort vs Nondiabetic control (Adjusted for sex/Gender)**

| Groups | Plasma Vitamin C Concentration | Mean Difference Diabetic Cohort) | (vs CI, min | CI, max | p-Values |
|---|---|---|---|---|---|
| Diabetic Cohort | 40.9 $\mu$M | NA | | | |
| Non-Diabetic Control | 53.1 $\mu$M | -5.85 | -17.647 | -5.293 | 0.00033 |
| Healthy Subgroup | 57.1 $\mu$M | -11.94 | -18.236 | -5.659 | 0.00025 |

Table 6

Fractional Excretion of Vitamin C in the diabetic cohort, nondiabetic controls and healthy subgroup. Numerical values for Figures 9A-9D

**A- Median FeVC in Diabetic cohort vs Nondiabetic control**

| | Non-Diabetic Control | Diabetic Cohort | Difference in Median | CI, min | CI, max | Shift in location |
|---|---|---|---|---|---|---|
| All Subjects | 0.00044 | 0.00032 | -0.00011 | -0.000644923 | 0.000232449 | -0.00003 |
| Plasma Vit C < MET | 0.0000 | 0.00015 | 0.00014 | 8.09526E-05 | 0.000242498 | -0.00006 |
| Plasma Vit C ≥ MET | 0.0010 | 0.0012 | 0.00011 | -0.000892115 | 0.001394258 | -0.00011 |

**B -Median FeVC Excretion in Diabetic cohort vs Healthy Subgroup**

| . | Healthy Subgroup | Diabetic Cohort | Difference in Median | CI, min | CI, max | Shift in location |
|---|---|---|---|---|---|---|
| All Subjects | 0.00046 | 0.00032 | -0.000093220 | 0.000644923 | 0.000232449 | -0.00003 |
| Plasma Vit C < MET | 0 | 0.00015 | -0.000324252 | 0.000269105 | 0.000541808 | -0.00024 |
| Plasma Vit C ≥ MET | 0.00046 | 0.00122 | -0.000604964 | 0.001327057 | 0.000163433 | -0.00017 |

Table 7

Clinical variables and their predictive association with vitamin C renal leak. Numerical values for Figure 10

| Demographics | | Log odds Ratio | CI, min | CI, max | p-Values |
|---|---|---|---|---|---|
| | Age | 0.023 | -0.0033 | 0.0497 | 0.08582 |
| | BMI | 0.097 | 0.0399 | 0.1543 | 0.00087 |
| | Ethnicity | | | | |
| | White/Caucasian | -0.182 | -1.6087 | 1.2441 | 0.80218 |
| | Black/African American | -0.323 | -1.7538 | 1.1083 | 0.65844 |
| | Hispanic | -0.511 | -3.0242 | 2.0026 | 0.69037 |
| Sex/Gender | Asian | | | | Reference |
| | Male | | | | Reference |
| Renal Function | Female | 0.105 | -0.6617 | 0.8724 | 0.78777 |
| | eGFR | -1.33 | -2.6762 | 0.4107 | 0.1503 |
| | PCR | 0.302 | 0.0197 | 0.5847 | 0.03602 |
| Glycemic Control | | | | | |
| | Glucose* | 0.12 | 0.047 | 0.194 | 0.00134 |
| | Hemoglobin A1C | 0.4 | 0.1709 | 0.6283 | 0.00062 |

*Values were multiplied by 10 for graphic representation. Abbreviation: CI, Confidence Intervals

## Claims

1. A method of assessing the vitamin C renal leak status of a human subject, wherein the human subject has fasted and not received any supplemental extraneous source of vitamin C for at least six hours and wherein the human subject undergoes an initial micturition after the at least six hours fasting, the initial micturition defining time 0, the method comprising:

   (a) collecting all subsequent urine output following time 0 as one or more urine voids from the human subject during a predefined timed interval, wherein the predefined timed interval encompasses a period of time of from 30 minutes to 24 hours and which begins at time 0,
   (b) obtaining a plasma sample from the human subject during the predefined timed interval, which begins at time 0,
   (c) providing no food or beverage items with high vitamin C content and no supplemental extraneous source of vitamin C throughout the predefined timed interval to the human subject,
   (d) assaying for the concentration of vitamin C in the plasma sample,

(e) assaying for the presence of vitamin C in the collected urine output, whereby:

(f) the presence of vitamin C in the collected urine output and a concentration of vitamin C in the plasma sample less than the Minimum Elimination Threshold (MET) indicates the presence of vitamin C renal leak in the human subject, wherein:

(g) the human subject is male, and the MET is between 32.5 $\mu$M and 43.5 $\mu$M, or

(h) the human subject is female, and the MET is between 35.5 $\mu$M and 51.0 $\mu$M.

2. The method of claim 1, wherein the human subject is male, and the MET is 38.1 $\mu$M or wherein the human subject is female, and the MET is 43.2 $\mu$M.

3. The method of claim 1 or 2 wherein the vitamin C in the collected urine output, in the plasma sample, or both is assayed using liquid chromatography optionally comprising coulometric electrochemical detection with high performance liquid chromatography or chromatography mass spectrometry (LCMS).

4. The method of any one of claims 1-3, wherein the human subject does not exhibit albuminuria, proteinuria or both albuminuria and proteinuria.

5. A method of identifying a clinical risk of renal impairment to a human subject comprising assessing vitamin C renal leak in a human subject in accordance with the method of any one of claims 1-4 , whereby an assessment that indicates the presence of vitamin C renal leak in the human subject identifies a clinical risk of renal impairment to the human subject.

6. The method of claim 5, wherein the human subject exhibits a family history of renal impairment or exhibits a genetic predisposition to develop renal impairment.

7. The method of claims 5 or 6, wherein the clinical risk of renal impairment is due to a disease or an intervention, procedure, or therapy, optionally wherein the intervention, procedure, or therapy comprises administration of a nephrotoxic agent, and optionally wherein the nephrotoxic agent comprises a contrast medium.

8. A method of screening for clinical risk of a disease in a human subject, the method comprising assessing vitamin C renal leak in a human subject in accordance with the method of any one of claims 1-4, whereby an assessment that indicates the presence of vitamin C renal leak in the human subject supports a conclusion that the human subject is at clinical risk of a disease.

9. A method of diagnosing a disease in a human subject, the method comprising assessing vitamin C renal leak in a human subject in accordance with the method of any one of claims 1-4, whereby an assessment that indicates the presence of vitamin C renal leak in the human subject supports a diagnosis of a disease in the human subject.

10. A method of screening for a risk of a disease complication or progression in a human subject, wherein the human subject is a patient with a disease, the method comprising assessing vitamin C renal leak in the human subject in accordance with the method of any one of claims 1-4 , whereby an assessment that indicates the presence of vitamin C renal leak in the human subject supports a conclusion that the human subject is at risk of a disease complication or progression, optionally wherein the disease complication or progression comprises renal impairment.

11. A method of monitoring disease state in a human subject, wherein the human subject is a patient with a disease, the method comprising obtaining a first assessment of vitamin C renal leak in the human subject and later obtaining a second assessment of vitamin C renal leak in the human subject, and then comparing the first assessment of renal vitamin C leak with the second assessment of renal vitamin C leak, wherein the renal vitamin C leak is assessed in accordance with the method of any one of claims 1-4 , and wherein:

(i) when the first assessment of vitamin C renal leak does not indicate the presence of renal vitamin C leak in the human subject and the second assessment of vitamin C renal leak indicates the presence of vitamin C renal leak in the human subject, a determination of disease progression in the human subject is made, or wherein

(ii) when the first assessment of vitamin C renal leak does not indicate the presence of renal vitamin C leak in the human subject and the second assessment of vitamin C renal leak does not indicate the presence of renal vitamin C leak in the human subject, a determination of disease stasis in the human subject is made, or wherein

(iii) when the first assessment of vitamin C renal leak indicates the presence of renal vitamin C leak in the human subject and the second assessment of vitamin C renal leak does not indicate the presence of vitamin C renal leak in the human subject, a determination of disease regression in the human subject is made, or wherein

(iv) when the first assessment of vitamin C renal leak indicates the presence of renal vitamin C leak in the human subject and the second assessment of vitamin C renal leak indicates the presence of renal vitamin C leak in the human subject, a determination of disease stasis in the human subject is made.

12. A method of predicting the response of a human subject to therapeutic treatment or prophylaxis, the method comprising assessing vitamin C renal leak in a human subject, wherein the human subject is a patient in need of therapeutic treatment of or prophylaxis from a disease, wherein the vitamin C renal leak is assessed in accordance with the method of any one of claims 1-4 , wherein an assessment that indicates the presence of vitamin C renal leak in the human subject results in a prediction that the human subject will be responsive to the therapeutic treatment or prophylaxis.

13. The method of claim 12, wherein the therapeutic treatment or prophylaxis comprises a procedure or agent bearing a risk of renal impairment.

14. The method of claim 12 or 13, wherein the therapeutic treatment or prophylaxis comprises the administration of an angiotensin converting enzyme (ACE) inhibitor, an aldosterone receptor blocker (ARB), an antihyperglycemic agent, a GLP-1 agonist. an SGLT-2 inhibitor, enzyme-replacement therapy, or vitamin C.

15. The method of any one of claims 8-14, wherein the disease is diabetes, an etiology involving the kidney, an autoimmune disease involving the kidney, a chronic urinary tract infection, Fabry disease, Retinopathy, cardiovascular disease, stroke, cancer, or HIV disease.

16. The method of claim 15, wherein the diabetes is type 1 diabetes or type 2 diabetes.

17. The method of any one of claims 1- 16, comprising further assessing fractional excretion of vitamin C (FeVC) in the human subject, the method comprising

(i) assaying for the concentration of vitamin C in the collected urine output,
(j) assaying for the volume of the collected urine output,
(k) assaying for the concentration of creatinine in the collected urine output, and
(l) assaying for the concentration of creatinine in the plasma sample,
(m) wherein the FeVC value is calculated as "A" according to the following equation:

$$A = \frac{\dfrac{[urine\ vitamin\ C\ concentration\ (\mu M)] * [urine\ volume\ (L)]}{[plasma\ vitamin\ C\ concentration\ (\mu M)]}}{\dfrac{[urine\ creatinine\ concentration\ (\mu M)] * [urine\ volume\ (L)]}{[plasma\ creatinine\ concentration\ (\mu M)]}}$$

**Patentansprüche**

1. Verfahren zur Bewertung des Status des Vitamin-C-Nierenlecks eines menschlichen Subjekts, wobei das menschliche Subjekt mindestens sechs Stunden lang gefastet hat und keine ergänzenden, äußeren Quellen von Vitamin C erhalten hat und wobei das menschliche Subjekt nach dem mindestens sechsstündigen Fasten eine anfängliche Miktion durchläuft, wobei die anfängliche Miktion den Zeitpunkt 0 definiert, wobei das Verfahren umfasst:

(a) Sammeln aller nachfolgenden Urinausscheidungen, die auf den Zeitpunkt 0 folgen, als eine oder mehrere Urinentleerungen von dem menschlichen Subjekt während eines vordefinierten Zeitintervalls, wobei das vordefinierte Zeitintervall eine Zeitspanne von 30 Minuten bis 24 Stunden umfasst und zum Zeitpunkt 0 beginnt,
(b) Gewinnung einer Plasmaprobe von dem menschlichen Subjekt während des vordefinierten Zeitintervalls, das zum Zeitpunkt 0 beginnt,
(c) keine Bereitstellung von Nahrungsmitteln oder Getränken mit hohem Vitamin-C-Gehalt und von ergänzenden, äußeren Quellen von Vitamin C während des gesamten vordefinierten Zeitintervalls für das menschliche Subjekt,
(d) Bestimmung der Konzentration von Vitamin C in der Plasmaprobe,
(e) Untersuchung auf das Vorhandensein von Vitamin C in der gesammelten Urinausscheidung, wobei:
(f) das Vorhandensein von Vitamin C in der gesammelten Urinausscheidung und eine Konzentration von Vitamin C in der Plasmaprobe, die unter dem minimalen Eliminationsschwellenwert (MES) liegt, das Vorhandensein eines Vitamin-C-Nierenlecks bei dem menschlichen Subjekt anzeigt, wobei:

(g) das menschliche Subjekt männlich ist und der MES zwischen 32,5 µM und 43,5 µM liegt, oder

(h) das menschliche Subjekt weiblich ist und der MES zwischen 35,5 µM und 51,0 µM liegt.

2. Verfahren nach Anspruch 1, wobei das menschliche Subjekt männlich ist und der MES 38,1 µM beträgt oder wobei das menschliche Subjekt weiblich ist und der MES 43,2 µM beträgt.

3. Verfahren nach Anspruch 1 oder 2, wobei das Vitamin C in der gesammelten Urinausscheidung, in der Plasmaprobe oder in beiden unter Verwendung von Flüssigkeitschromatographie, die optional eine coulometrische elektrochemische Detektion mit Hochleistungsflüssigkeitschromatographie oder Chromatographie-Massenspektrometrie (LC-MS) umfasst, bestimmt wird.

4. Verfahren nach einem der Ansprüche 1 bis 3, wobei das menschliche Subjekt keine Albuminurie, Proteinurie oder sowohl Albuminurie als auch Proteinurie aufweist.

5. Verfahren zur Identifizierung eines klinischen Risikos einer Nierenbeeinträchtigung bei einem menschlichen Subjekt, dass die Bewertung des Vitamin-C-Nierenlecks bei einem menschlichen Subjekt gemäß dem Verfahren nach einem der Ansprüche 1 bis 4 umfasst, wobei eine Bewertung, die das Vorhandensein eines Vitamin-C-Nierenlecks bei dem menschlichen Subjekt anzeigt, auf ein klinisches Risiko einer Nierenbeeinträchtigung bei dem menschlichen Subjekt hinweist.

6. Verfahren nach Anspruch 5, wobei das menschliche Subjekt eine familiäre Vorgeschichte von Nierenbeeinträchtigung aufweist oder eine genetische Veranlagung zur Entwicklung von Nierenbeeinträchtigung aufweist.

7. Verfahren nach Anspruch 5 oder 6, wobei das klinische Risiko einer Nierenbeeinträchtigung auf eine Krankheit oder einen Eingriff, ein Verfahren oder eine Therapie zurückzuführen ist, wobei der Eingriff, das Verfahren oder die Therapie optional die Verabreichung eines nephrotoxischen Mittels umfasst, und wobei das nephrotoxische Mittel optional ein Kontrastmittel umfasst.

8. Verfahren zum Screening eines klinischen Risikos einer Krankheit bei einem menschlichen Subjekt, wobei das Verfahren das Beurteilen des Vitamin-C-Nierenlecks bei einem menschlichen Subjekt gemäß dem Verfahren nach einem der Ansprüche 1 bis 4 umfasst, wobei eine Bewertung, die das Vorhandensein eines Vitamin-C-Nierenlecks bei dem menschlichen Subjekt anzeigt, eine Schlussfolgerung, dass das menschliche Subjekt ein klinisches Risiko für eine Krankheit aufweist, unterstützt.

9. Verfahren zur Diagnose einer Krankheit in einem menschlichen Subjekt, wobei das Verfahren die Bewertung des Vitamin-C-Nierenlecks bei einem menschlichen Subjekt gemäß dem Verfahren nach einem der Ansprüche 1-4 umfasst, wobei eine Bewertung, die das Vorhandensein eines Vitamin-C-Nierenlecks bei dem menschlichen Subjekt anzeigt, eine Diagnose einer Krankheit in dem menschlichen Subjekt stützt.

10. Verfahren zur Untersuchung eines Risikos einer Komplikation oder eines Fortschreitens eine Erkrankung bei einem menschlichen Subjekt, wobei das menschliche Subjekt ein Patient mit einer Krankheit ist, wobei das Verfahren die Bewertung eines Vitamin-C-Nierenlecks bei dem menschlichen Subjekt gemäß dem Verfahren nach einem der Ansprüche 1 bis 4 umfasst, wobei eine Bewertung, die das Vorhandensein eines Vitamin-C-Nierenlecks bei dem menschlichen Subjekt anzeigt, eine Schlussfolgerung unterstützt, dass das menschliche Subjekt einem Risiko einer Komplikation oder eines Fortschreitens einer Erkrankung ausgesetzt ist, wobei die Komplikation oder das Fortschreitens der Erkrankung optional eine Nierenbeeinträchtigung umfasst.

11. Verfahren zur Überwachung des Krankheitszustandes in einem menschlichen Subjekt, wobei das menschliche Subjekt ein Patient mit einer Krankheit ist, wobei das Verfahren das Erhalten einer ersten Bewertung des Vitamin-C-Nierenlecks bei dem menschlichen Subjekt und das spätere Erhalten einer zweiten Bewertung des Vitamin-C-Nierenlecks bei dem menschlichen Subjekt und folglich das Vergleichen der ersten Bewertung des Vitamin-C-Nierenlecks mit der zweiten Bewertung des Vitamin-C-Nierenlecks umfasst, wobei das Vitamin-C-Nierenleck gemäß dem Verfahren nach einem der Ansprüche 1 bis 4 bewertet wird, und wobei:

(i) wenn die erste Bewertung des Vitamin-C-Nierenlecks nicht auf das Vorhandensein eines Vitamin-C-Nierenlecks bei dem menschlichen Subjekt hinweist und die zweite Bewertung des Vitamin-C-Nierenlecks auf das Vorhandensein eines Vitamin-C-Nierenlecks bei dem menschlichen Subjekt hinweist, eine Bestimmung des Krankheitsverlaufs bei dem menschlichen Subjekt vorgenommen wird, oder wobei

(ii) wenn die erste Bewertung des Vitamin-C-Nierenlecks nicht auf das Vorhandensein eines Vitamin-C-Nierenlecks bei dem menschlichen Subjekt hinweist und die zweite Bewertung des Vitamin-C-Nierenlecks nicht auf das Vorhandensein eines Vitamin-C-Nierenlecks bei dem menschlichen Subjekt hinweist, eine Bestimmung der Krankheitsstase bei dem menschlichen Subjekt vorgenommen wird, oder wobei

(iii) wenn die erste Bewertung des Vitamin-C-Nierenlecks das Vorhandensein eines Vitamin-C-Nierenlecks bei dem menschlichen Subjekt anzeigt und die zweite Bewertung des Vitamin-C-Nierenlecks nicht das Vorhandensein eines Vitamin-C-Nierenlecks bei dem menschlichen Subjekt anzeigt, eine Bestimmung der Krankheitsregression bei dem menschlichen Subjekt vorgenommen wird, oder wobei

(iv) wenn die erste Bewertung des Vitamin-C-Nierenlecks das Vorhandensein eines Vitamin-C-Nierenlecks bei dem menschlichen Subjekt anzeigt und die zweite Bewertung des Vitamin-C-Nierenlecks das Vorhandensein eines Vitamin-C-Nierenlecks bei dem menschlichen Subjekt anzeigt, wird eine Bestimmung der Krankheitsstase bei dem menschlichen Subjekt vorgenommen.

12. Verfahren zur Vorhersage des Ansprechens eines menschlichen Subjekts auf eine therapeutische Behandlung oder Prophylaxe, wobei das Verfahren das Beurteilen des Vitamin-C-Nierenlecks bei einem menschlichen Subjekt umfasst, wobei das menschliche Subjekt ein Patient ist, der eine therapeutische Behandlung oder Prophylaxe einer Krankheit benötigt, wobei das Vitamin-C-Nierenleck gemäß dem Verfahren nach einem der Ansprüche 1-4 beurteilt wird, wobei eine Bewertung, die das Vorhandensein eines Vitamin-C-Nierenlecks bei dem menschlichen Subjekt anzeigt, zu einer Vorhersage führt, dass das menschliche Subjekt auf die therapeutische Behandlung oder Prophylaxe ansprechen wird.

13. Verfahren nach Anspruch 12, wobei die therapeutische Behandlung oder Prophylaxe ein Verfahren oder einen Wirkstoff umfasst, das bzw. der das Risiko einer Nierenschädigung mit sich bringt.

14. Verfahren nach Anspruch 12 oder 13, wobei die therapeutische Behandlung oder Prophylaxe die Verabreichung eines Angiotensin-Converting-Enzyme-(ACE)-Hemmers, eines Aldosteron-Rezeptorblockers (ARB), eines antihyperglykämischen Mittels, eines GLP-1-Agonisten, eines SGLT-2-Hemmers, einer Enzymersatztherapie oder von Vitamin C umfasst.

15. Verfahren nach einem der Ansprüche 8-14, wobei es sich bei der Krankheit um Diabetes, eine die Niere betreffende Ätiologie, eine die Niere betreffende Autoimmunerkrankung, eine chronische Harnwegsinfektion, Morbus Fabry, eine Retinopathie, eine Herz-Kreislauf-Erkrankung, einen Schlaganfall, Krebs oder eine HIV-Erkrankung handelt.

16. Verfahren nach Anspruch 15, wobei Diabetes Typ-1-Diabetes oder Typ-2-Diabetes ist.

17. Verfahren nach einem der Ansprüche 1 bis 16, das ferner die Bestimmung der fraktionierten Ausscheidung von Vitamin C (FAVC) bei dem menschlichen Subjekt umfasst, wobei das Verfahren umfasst

(i) Bestimmung der Konzentration von Vitamin C in den gesammelten Urinausscheidungen,
(j) die Bestimmung des Volumens der gesammelten Urinausscheidung,
(k) Bestimmung der Konzentration von Kreatinin in der gesammelten Urinausscheidung, und
(l) Bestimmung der Konzentration von Kreatinin in der Plasmaprobe,
(m) wobei der FAVC-Wert als "A" nach der folgenden Gleichung berechnet wird:

$$A = \frac{\dfrac{[Konzentration\ von\ Vitamin\ C\ im\ Urin\ (\mu M)] * [Urinvolumen\ (L)]}{[Konzentration\ von\ Vitamin\ C\ im\ Plasma\ (\mu M)]}}{\dfrac{[Konzentration\ von\ Kreatinin\ im\ Urin\ (\mu M)] * [Urinvolumen\ (L)]}{[Konzentration\ von\ Kreatinin\ im\ Plasma\ (\mu M)]}}$$

**Revendications**

1. Procédé d'évaluation de l'état de perte rénale de vitamine C d'un sujet humain, sachant que le sujet humain a jeûné et n'a reçu aucune source externe supplémentaire de vitamine C pendant au moins six heures et sachant que le sujet humain effectue une miction initiale après les au moins six heures de jeûne, la miction initiale définissant le temps 0, le procédé comprenant :

(a) la collecte de toute la diurèse subséquente après le temps 0 en tant qu'une ou plusieurs émissions urinaires provenant du sujet humain pendant un intervalle de temps prédéfini, sachant que l'intervalle de temps prédéfini comprend une période de 30 minutes à 24 heures et qui commence au temps 0,
(b) l'obtention d'un échantillon de plasma provenant du sujet humain pendant l'intervalle de temps prédéfini, qui commence au temps 0,
(c) le fait de ne fournir au sujet humain aucun aliment ou boisson à teneur élevée en vitamine C ni aucune source externe supplémentaire de vitamine C tout au long de l'intervalle de temps prédéfini,
(d) l'analyse de la concentration de vitamine C dans l'échantillon de plasma,
(e) l'analyse de la présence de vitamine C dans la diurèse collectée, en vertu de quoi :
(f) la présence de vitamine C dans la diurèse collectée et une concentration de vitamine C dans l'échantillon de plasma inférieure au seuil d'élimination minimum (MET) indiquent la présence d'une perte rénale de vitamine C chez le sujet humain, sachant que :
(g) le sujet humain est de sexe masculin, et le MET est compris entre 32,5 $\mu$M et 43,5 $\mu$M, ou
(h) le sujet humain est de sexe féminin, et le MET est compris entre 35,5 $\mu$M et 51,0 $\mu$M.

2. Le procédé de la revendication 1, sachant que le sujet humain est de sexe masculin, et le MET est de 38,1 $\mu$M ou sachant que sujet humain est de sexe féminin, et le MET est de 43,2 $\mu$M.

3. Le procédé de la revendication 1 ou 2, sachant que la vitamine C dans la diurèse collectée, dans l'échantillon de plasma, ou les deux, est analysée par chromatographie en phase liquide comprenant facultativement une détection coulométrique électrochimique avec chromatographie en phase liquide à haute performance ou chromatographie-spectrométrie de masse (LCMS).

4. Le procédé de l'une quelconque des revendications 1 à 3, sachant que le sujet humain ne présente pas d'albuminurie, de protéinurie ou à la fois d'albuminurie et de protéinurie.

5. Procédé d'identification d'un risque clinique d'insuffisance rénale pour un sujet humain comprenant l'évaluation d'une perte rénale de vitamine C chez un sujet humain selon le procédé de l'une quelconque des revendications 1 à 4, en vertu de quoi une évaluation indiquant la présence d'une perte rénale de vitamine C chez le sujet humain identifie un risque clinique d'insuffisance rénale pour le sujet humain.

6. Le procédé de la revendication 5, sachant que le sujet humain présente des antécédents familiaux d'insuffisance rénale ou présente une prédisposition génétique de développer une insuffisance rénale.

7. Le procédé de la revendication 5 ou 6, sachant que le risque clinique d'insuffisance rénale est dû à une maladie ou une intervention, une procédure, ou une thérapie, facultativement sachant que l'intervention, la procédure, ou la thérapie comprend l'administration d'un agent néphrotoxique, et facultativement sachant que l'agent néphrotoxique comprend un produit de contraste.

8. Procédé de dépistage d'un risque clinique de maladie chez un sujet humain, le procédé comprenant l'évaluation d'une perte rénale de vitamine C chez un sujet humain selon le procédé de l'une quelconque des revendications 1 à 4, en vertu de quoi une évaluation indiquant la présence d'une perte rénale de vitamine C chez le sujet humain appuie une conclusion selon laquelle le sujet humain présente un risque clinique de maladie.

9. Procédé de diagnostic d'une maladie chez un sujet humain, le procédé comprenant l'évaluation d'une perte rénale de vitamine C chez un sujet humain selon le procédé de l'une quelconque des revendications 1 à 4, en vertu de quoi une évaluation indiquant la présence d'une perte rénale de vitamine C chez le sujet humain appuie un diagnostic d'une maladie chez le sujet humain.

10. Procédé de dépistage d'un risque de complication ou de progression de maladie chez un sujet humain, sachant que le sujet humain est un patient atteint d'une maladie, le procédé comprenant l'évaluation d'une perte rénale de vitamine C chez le sujet humain selon le procédé de l'une quelconque des revendications 1 à 4, en vertu de quoi une évaluation indiquant la présence d'une perte rénale de vitamine C chez le sujet humain appuie une conclusion selon laquelle le sujet humain présente un risque de complication ou de progression de maladie, facultativement sachant que la complication ou la progression de maladie comprend une insuffisance rénale.

**11.** Procédé de surveillance d'un état de maladie chez un sujet humain, sachant que le sujet humain est un patient atteint d'une maladie, le procédé comprenant l'obtention d'une première évaluation d'une perte rénale de vitamine C chez le sujet humain et ultérieurement l'obtention d'une seconde évaluation d'une perte rénale de vitamine C chez le sujet humain, et ensuite la comparaison de la première évaluation d'une perte rénale de vitamine C avec la seconde évaluation d'une perte rénale de vitamine C, sachant que la perte rénale de vitamine C est évaluée selon le procédé de l'une quelconque des revendications 1 à 4, et sachant que :

(i) lorsque la première évaluation d'une perte rénale de vitamine C n'indique pas la présence d'une perte rénale de vitamine C chez le sujet humain et la seconde évaluation d'une perte rénale de vitamine C indique la présence d'une perte rénale de vitamine C chez le sujet humain, une détermination de progression de maladie chez le sujet humain est faite, ou sachant que

(ii) lorsque la première évaluation d'une perte rénale de vitamine C n'indique pas la présence d'une perte rénale de vitamine C chez le sujet humain et la seconde évaluation d'une perte rénale de vitamine C n'indique pas la présence d'une perte rénale de vitamine C chez le sujet humain, une détermination de stase de maladie chez le sujet humain est faite, ou sachant que

(iii) lorsque la première évaluation d'une perte rénale de vitamine C indique la présence d'une perte rénale de vitamine C chez le sujet humain et la seconde évaluation d'une perte rénale de vitamine C n'indique pas la présence d'une perte rénale de vitamine C chez le sujet humain, une détermination de régression de maladie chez le sujet humain est faite, ou sachant que

(iv) lorsque la première évaluation d'une perte rénale de vitamine C indique la présence d'une perte rénale de vitamine C chez le sujet humain et la seconde évaluation d'une perte rénale de vitamine C indique la présence d'une perte rénale de vitamine C chez le sujet humain, une détermination de stase de maladie chez le sujet humain est faite.

**12.** Procédé de prédiction de la réponse d'un sujet humain à un traitement thérapeutique ou une prophylaxie, le procédé comprenant l'évaluation d'une perte rénale de vitamine C chez un sujet humain, sachant que le sujet humain est un patient qui nécessite un traitement thérapeutique pour une maladie ou une prophylaxie contre une maladie, sachant que la perte rénale de vitamine C est évaluée selon le procédé de l'une quelconque des revendications 1 à 4, sachant qu'une évaluation indiquant la présence d'une perte rénale de vitamine C chez le sujet humain aboutit à une prédiction selon laquelle le sujet humain répondra au traitement thérapeutique ou à la prophylaxie.

**13.** Le procédé de la revendication 12, sachant que le traitement thérapeutique ou la prophylaxie comprend une procédure ou un agent comportant un risque d'insuffisance rénale.

**14.** Le procédé de la revendication 12 ou 13, sachant que le traitement thérapeutique ou la prophylaxie comprend l'administration d'un inhibiteur de l'enzyme de conversion de l'angiotensine (ACE), d'un bloqueur du récepteur de l'aldostérone (ARB), d'un agent antihyperglycémique, d'un agoniste de GLP-1, d'un inhibiteur de SGLT-2, d'une thérapie de substitution enzymatique, ou de vitamine C.

**15.** Le procédé de l'une quelconque des revendications 8 à 14, sachant que la maladie est du diabète, une étiologie impliquant le foie, une maladie auto-immune impliquant le foie, une infection chronique des voies urinaires, la maladie de Fabry, une rétinopathie, une maladie cardiovasculaire, un AVC, un cancer, ou une maladie du VIH.

**16.** Le procédé de la revendication 15, sachant que le diabète est un diabète de type 1 ou un diabète de type 2.

**17.** Le procédé de l'une quelconque des revendications 1 à 16, comprenant en outre l'évaluation d'une excrétion fractionnelle de vitamine C (FeVC) chez le sujet humain, le procédé comprenant

(i) l'analyse de la concentration de vitamine C dans la diurèse collectée,
(j) l'analyse du volume de la diurèse collectée,
(k) l'analyse de la concentration de créatinine dans la diurèse collectée, et
(l) l'analyse de la concentration de créatinine dans l'échantillon de plasma,
(m) sachant que la valeur FeVC est calculée comme « A » selon l'équation suivante :

$$A = \frac{\frac{[concentration\ de\ vitamine\ C\ dans\ l'urine\ (\mu M)]\ *\ [volume\ d'urine\ (L)]}{[concentration\ de\ vitamine\ C\ dans\ le\ plasma\ (\mu M)]}}{\frac{[concentration\ de\ créatinine\ dans\ l'urine\ (\mu M)]\ *\ [volume\ d'urine\ (L)]}{[concentration\ de\ créatinine\ dans\ le\ plasma\ (\mu M)]}}$$

Fig. 1A

Fig. 1B

Fig. 2A

Fig. 2B

Fig. 2C

Fig. 2D

Fig. 3A

Renal Leak Study: Fabry Disease

Fig. 3B

| Control Group | Fabry Disease Cohort | Odds Ratio | | p-value |
|---|---|---|---|---|
| 6% | 52% | 16.3 | | <0.001 |

Fig. 3C

Fig. 3D

Fig. 3E

| | Difference in Median | | p-value | Estimated Shift |
|---|---|---|---|---|
| All Subjects | 0.018 | | 0.010 | 0.004 |
| Plasma Vit C <MET | 0.009 | | <0.001 | 0.009 |
| Plasma Vit C <MET | 0.024 | | 0.012 | 0.020 |

-0.01    0    0.025

Estimated difference in group median FeVC

Fig. 3F

EP 4 348 268 B1

Clinical and Lab Covariates

| | Estimated shift in median | p-Values unadjusted [Adjusted within-group] |
|---|---|---|
| **Demographics** | | |
| Age | | 0.244 [0.322] |
| BMI | | 0.322 [0.322] |
| **Renal Function** | | |
| eGFR | | <0.001 [<0.001] |
| PCR* | | <0.001 [<0.001] |
| Protein Excretion* | | <0.001 [0.002] |
| ACE/ARB/Diuretics | | 0.060 [0.060] |
| **Lab Predictors** | | |
| Glucose | | 0.088 [0.265] |
| AST | | 0.016 [0.081] |
| ALT | | 0.331 [0.390] |
| Alkaline Phosphatase | | 0.390 [0.390] |
| Hemoglobin | | 0.002 [0.011] |

-0.001          0          0.001

Estimated shift in median

Fig. 4

EP 4 348 268 B1

Renal Leak Prevalence vs Plasma Vitamin C

Fig. 5A

| | Plasma Vitamin C | | | Renal leak at 1h | |
| --- | --- | --- | --- | --- | --- |
| | Mean (µM) | Confidence Interval | Proportion of leak (%) | | Confidence Interval |
| Healthy | 38.9 | 31.79 – 46.01 | 5.9 | | 0.0072 – 0.1968 |
| Fabry | 36.5 | 29.78 – 43.16 | 51.5 | | 0.335 – 0.692 |

Fig. 5B

| | Healthy | Fabry | Odds Ratio | | p-value |
|---|---------|-------|------------|---|---------|
| 1h | 6% | 52% | 16.3 | | <0.001 |
| 24h | 3% | 48 | 29.6 | | <0.001 |

Odds Ratio: 1    20    40

Fig. 5C

Fig. 6A

Gulo mouse spot urine

Fig. 6B

Fig. 6C

Fig. 6D

Fig. 7

Fig. 8A

Fig. 8B

Fig. 8C

EP 4 348 268 B1

| Groups | Renal leak prevalence | Odds Ratio (vs Diabetic cohort) | | p-Values |
|---|---|---|---|---|
| Diabetes Cohort | 32.9% | -- | | |
| Nondiabetic Controls | 8.8% | 5.07 | | <0.001 |
| Healthy Control | 0.0% | Undefined | | <0.001 |

Odds Ratio (0 1 5 10)

Fig. 8D

EP 4 348 268 B1

| Groups | Mean Plasma Vitamin C Concentration | Mean Difference (vs Diabetes Cohort) | | p-Values Unadjusted |
|---|---|---|---|---|
| Diabetes Cohort | 40.9 µM | — | | |
| Nondiabetic Controls | 53.1 µM | -12.20 | | <0.001 |
| Healthy Control | 56.3 µM | -15.48 | | <0.001 |

-20    -10    0    5

Estimated Shift in mean Plasma Vitamin C

Fig. 8E

Fig. 9A

Fig. 9B

FeVC in Healthy Subgroup

Fig. 9C

## Median FeVC in Diabetic cohort vs Nondiabetic control

| | Difference in Median | | Shift in location |
|---|---|---|---|
| All Subjects | -0.00011 | | -0.00003 |
| Subjects with Plasma Vit C < MET | 0.00014 | | -0.00006 |
| Subjects with Plasma Vit C > MET | 0.00011 | | -0.00011 |

-0.001　　0　　0.001
Estimated difference in group median FeVC

## Median FeVC in Diabetic cohort vs Healthy control

| | Difference in Median | | Shift in location |
|---|---|---|---|
| All Subjects | -0.00009 | | -0.00003 |
| Subjects with Plasma Vit C < MET | 0.00032 | | -0.00024 |
| Subjects with Plasma Vit C > MET | -0.0006 | | -0.00017 |

-0.001　　0　　0.001
Estimated difference in group median FeVC

Fig. 9D

EP 4 348 268 B1

## Clinical variables predictive of vitamin C renal leak; all subjects

| Demographics | | |
|---|---|---|
| Age | | 0.086 |
| BMI | | <0.001 |
| Ethnicity | | |
| White/Caucasian | | 0.802 |
| Black/African American | | 0.658 |
| Hispanic | | 0.690 |
| Asian | | Reference |
| **Sex/Gender** | | |
| Male | | Reference |
| Female | | 0.788 |
| **Renal Function** | | |
| eGFR | | 0.150 |
| PCR | | 0.036 |
| **Glycemic Control** | | |
| Glucose* | | 0.001 |
| Hemoglobin A1C | | <0.001 |

0           1           2

Odds ratio

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JACKSON et al.** *Journal of Orthomolecular Medicine*, 2005, vol. 20 (4)